(19) Europäisches Patentamt
European Patent Office
Office européen des brevets

(11) Publication number : **0 539 209 A1**

(12) **EUROPEAN PATENT APPLICATION**

(21) Application number : **92309695.2**

(22) Date of filing : **22.10.92**

(51) Int. Cl.[5] : **C07D 221/10**, C07D 223/32, C07D 221/16, C07D 223/14, A61K 31/47, A61K 31/435

(30) Priority : **24.10.91 US 782253**
**22.11.91 US 795456**
**03.09.92 PCT/US92/07314**

(43) Date of publication of application :
**28.04.93 Bulletin 93/17**

(84) Designated Contracting States :
**AT BE CH DE DK ES FR GB GR IE IT LI LU MC NL PT SE**

(71) Applicant : **THE UPJOHN COMPANY**
**301 Henrietta Street**
**Kalamazoo, Michigan 49001 (US)**

(72) Inventor : **Romero, Arthur Glenn**
**2721 Lake Chevy Chase Drive**
**Kalamazoo, Michigan 49008 (US)**

(74) Representative : **Perry, Robert Edward**
**GILL JENNINGS & EVERY, Broadgate House,**
**7 Eldon Street**
**London EC2M 7LH (GB)**

(54) **Benzo-isoquinoline derivatives and analogs and their use in therapeutics.**

(57) The present invention encompasses : 1) compounds of formula I below,

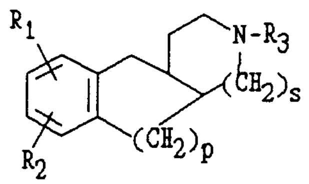

Formula I

wherein p and s are independent and may be either 1 or 2,
wherein $R_1$ is -H, -Halo, -CN, $-CO_2H$, $-CO_2R_{1-1}$, $-CONH_2$, $-CONHR_{1-1}$, $-CON(R_{1-1})_2$, -SH,- $SR_{1-1}$, $-SO_2R_{1-1}$, $SO_2NH_2$, $-SO_2NHR_{1-1}$, $-SO_2N(R_{1-1})_2$, $-OR_{1-1}$, $-OSO_2CF_3$, $-OSO_2R_{1-1}$, $-NH_2$, $-NHR_{1-1}$, or $-N(R_{1-1})_2$ ; wherein $R_{1-1}$ is -H, -($C_1$-$C_8$ alkyl), -($C_1$-$C_8$ alkenyl), -($C_3$-$C_{10}$ cycloalkyl), -($C_6$ aryl), -5 or 6 member heterocyclics, -($C_1$-$C_8$ alkyl)-(5 or 6 member heterocyclics), wherein $R_2$ is -H, -Halo, -CN, $-CF_3$, -SH, or $-SR_{2-1}$ ; wherein $R_{2-1}$ = $R_{1-1}$, wherein $R_3$ is -H, -($C_1$-$C_8$ alkyl), -($C_1$-$C_8$ alkenyl), -($C_6$ aryl), -($C_3$-$C_{10}$ cycloalkyl), -(5 or 6 member heterocyclics), -($C_1$-$C_8$ alkyl)-5 or 6 member heterocyclics), wherein $R_{3-1}$ = $R_{1-1}$ or a pharmacologically acceptable salt thereof. 2. A pharmaceutical composition consisting essentially of a pharmaceutically acceptable carrier and an effective amount of a compound of formula I. 3. A method of treating central nervous system disorders, associated with serotonin and or dopamine receptor activity comprising : administering an effective amount of a compound of formula I to a patient in need thereof. The compounds of this invention possess selective pharmacological properties and are useful in treating central nervous system disorders.

EP 0 539 209 A1

## FIELD OF THE INVENTION

The present invention relates to novel compositions of matter. More particularly, the present invention relates to new isoquinoline and pyridine derivatives, processes for preparing these compounds, and pharmaceutical compositions containing these compounds. The present invention further relates to the use of these compounds in the treatment of certain psychiatric disorders, such as anxiety, depression, sexual dysfunction schizophrenia and parkinsonism.

## BACKGROUND OF THE INVENTION

Psychiatric diseases are thought to be due to dysfunctions in monoaminergic neuronal systems, particularly those involving serotonin (5-HT) and dopamine (DA).

### Serotonin (5-$HT_{1A}$)

Anxiety is associated with increased activity in 5-HT systems. In animals where 5-HT has been depleted, benzodiazepine anxiolgics are not active in anti-anxiety assays that they otherwise are effective in. Serotonin neurons have autoreceptors that, when activated by agonists, depress firing rates of 5-HT cells. These receptors are of the 5-$HT_{1A}$ subtype. Because they depress 5-HT neuronal activity, it can be expected that 5-$HT_{1A}$ agonists will be an effective anxiolytic. Clinically, 5-$HT_{1A}$ agonists have demonstrated anxiolytic properties. The drug buspirone is the only currently available marketed 5-$HT_{1A}$ agonist having anxiolytic activity. This compound antagonizes dopamine receptors at the same dose it stimulates 5-$HT_{1A}$ receptors. A similar drug, gepirone, also has dopamine antagonist properties. These dopamine antagonist properties reduce the clinical utility of these compounds because long term treatment with dopamine antagonists can produce tardive dyskinesia.

Depression is a psychiatric condition thought to be associated with decreased 5-HT release. Most antidepressants potentiate the effects of 5-HT by blocking the termination of activity through re-uptake into nerve terminals. Since some 5-$HT_{1A}$ receptors are activated postsynaptically by 5-HT, 5-$HT_{1A}$ agonists may also be anti-depressants. Since the postsynaptic 5-$HT_{1A}$ receptor may be less sensitive than the autoreceptor, high doses of 5-$HT_{1A}$ agonists, particularly very effective ones (i.e., those causing greater stimulation of the 5-$HT_{1A}$ receptor, a parameter referred to as "efficacy"), can be expected to be effective anti-depressants. Gepirone has already been demonstrated to have ameliorative effects on some depressive end points in some patients.

Serotonin is also involved in the regulation of feeding and sexual behavior and in cardiovascular regulation. Thus, 5-$HT_{1A}$ agonists may be useful in treating overeating and sexual dysfunction. These compounds have been shown to alter feeding and sexual behavior in animals. 5-$HT_{1A}$ agonists are also known to depress sympathetic nerve discharge and thus lower blood pressure. Thus, they may be useful in treating hypertension, congestive heart failure by reducing cardiovascular afterload and heart attack by removing sympathetic drive to the heart.

### Dopamine (DA)

Schizophrenia is thought to be due to hyperactivity in DA systems. Thus, currently available antipsychotics are DA antagonists. Dopamine autoreceptors depress DA neuron firing rates, DA synthesis and release. Thus DA autoreceptor agonists can also be expected to be antipsychotics. DA agonists are also useful for treating Parkinsonism, a disease caused by degeneration of DA neurons, and hyperprolactinemia, since DA agonists depress prolactin release.

Dopamine autoreceptor antagonists are a new class of drug that increase release of DA by releasing the DA neuron from autoreceptor control. Thus, these drugs can be expected to be useful in conditions treatable with amphetamine and other similar stimulants which directly release DA. Dopamine autoreceptor agonists are expected to be much milder stimulants than amphetamines because they simply increase the release associated with the normal DA activity, by releasing the cell from autoreceptor control, rather than directly releasing DA. Thus, DA autoreceptor antagonists can be expected to be useful in treating overeating, attention deficit disorders, psychiatric, cognitive and motor retardation in demented and elderly patients, and in treating nausea and dizziness.

Drugs acting on central DA transmission are clinically effective in treating a variety of central nervous system disorders such as parkinsonism, schizophrenia, and manic-depressive illness. In parkinsonism, for example, the nigro-neostriatal hypofunction can be restored by an increase in postsynaptic DA receptor stimulation. In schizophrenia, the condition can be normalized by achieving a decrease in postsynaptic DA receptor stimulation. Classical antipsychotic agents directly block the postsynaptic DA receptor. The same effect can be achieved by inhibition of intraneuronal presynaptic events essential for the maintenance of adequate neurotransmission, transport mechanism and transmitter synthesis.

In recent years a large body of pharmacological, biochemical and electrophysical evidence has provided considerable support in favor of the existence of a specific population of central autoregulatory DA receptors located in the dopaminergic neuron itself. These receptors are part of a homeostatic mechanism that modulates nerve impulse low and transmitter synthesis and regulates the amount of DA released from the nerve endings.

Direct DA receptor agonists, like apomorphine, are able to activate the DA autoreceptors as well as the post synaptic DA receptors. The effects of autoreceptor stimulation appear to predominate when apomorphine is administered at low doses, whereas at higher doses the attenuation of DA transmission is outweighed by the enhancement of postsynaptic receptor stimulation. The anti-psychotic and anti-dyskinetic effects in man of low doses of apomorphine are likely due to the autoreceptor-stimulator properties of this DA receptor agonist. This body of knowledge indicates DA receptor stimulants with a high selectivity for central nervous DA autoreceptors would be valuable in treating psychiatric disorders. The compounds of the present invention have a variety of effects at 5-$HT_{1A}$ and DA receptors, and offer a variety of utilities associated with those activities.

DESCRIPTION OF RELATED ART

The compounds of this invention may be described as having tricyclic moieties, or rings. These rings, have been labeled A, B, C, in Formula I, below, in the summary of invention section. The tricyclic moieties may be designated by the number of atoms in each ring. Thus, a 6-5-7 ring system would have 6 atoms in the A ring, 5 atoms in the B ring, and 7 atoms in the C ring.

The compounds of this invention have a 6-6-6 ring system. One of the atoms in the C - ring is nitrogen. A 6-6-6 ring system where the A ring is aromatic that contains a 6-atom C ring which contains nitrogen might also be called a benzo-quinoline structure. Included below are documents that represent the 6-6-6 system, "SET A, " in addition to other systems. These other systems are grouped and listed below for the sake of completeness. The first two references cited in "SET A" are considered most relevant to this disclosure.

Some documents cited below describe compounds that belong in more than one group, but generally the following groups are observed: The first group, SET A, contains 6-6-6 ring systems or benzo-quinolone structures; the second group, SET B, contains 6-5-6 ring systems or indenopyridine structures; the third group, SET C, contains 6-6-5 ring systems or benzo-indolines; the fourth group, SET D, contains 6-5-5 ring systems or indeno-pyrroles, and the fifth group SET E, contains 6-7-5 ring systems or benzo-cyclohepta-pyridines.

These documents are described below:

SET A (6-6-6 ring systems)

European Patent Application, EP 410,535-A, application published 30 January 1991, Derwent No. 91-030926/05, discloses octahydrobenzisoquinoline derivatives as antipsychotic agents. Published PCT application, international publication number WO 90/06927, PCT/US89/05512, international publication date, 28 June 1990, Derwent No. 90-224487/29, discloses hexahydroindeno[1,2-c]pyrroles, hexahydroindenopyrodines and hexahydrobenz[e]isoindoles as selective 5-HT receptor agents; United States Patent, US 4,341,786, issued 27 July 1982, discloses octahydrobenzo[f]quinoline compounds. Great Britain Patent, GB 1,596,170, published 19 August 1981, Derwent No. 55370A/31 discloses hexahydro-benz[f]isoquinolines. Great Britain Patent Application, GB 2,126,584-A, application published 28 March 1984, Derwent No. 84-077373/13, discloses octahydrobenz[f]isoquinolines as psychotropic and analgesic agents. Great Britain Patent, GB 2138-815-A, published 27 April 1983, Derwent No. 84-271,830 discloses benzo[f]quinolines. German Patent, DE 2,801,576, Derwent No. 55370/31, discloses hexahydrobenz[f]isoquinolines.

SET B (6-5-6 ring systems)

Soviet Union Patent, SU 327193, Derwent No. 63503T-B, discloses isoquinolines; and German Patent, DE 2,501,930, Derwent No. 58024X/31, discloses hexahydro-indeno pyridines as antidepressants.

SET C (6-6-5 ring systems)

Published PCT application, WO91/13872, published 19 September 1991, international application No. PCT/US91/00117 discloses heterocyclic indole compounds. Published PCT application WO91/11435, published 8 August 1991, international application No. PCT/US91/00018 discloses benz(e)indole heterocyclic compounds. Published PCT application WO91/00856, published 24 january 1991, international application No. PCT/US90/03551 discloses carbocyclic-2-amino tetralin derivatives. United States Patent, US 4,618,683, issued 21 October 1986, Derwent No. 86-298,374/45 discloses tetrahydro-benzo[e]indolines. European Patent, EP 95-666-A, published 1 june 1980, Derwent No. 83-840180, discloses tetra:hydro-benzo-isoindolines. Netherland Patent, NL 7216762, published 8 December 1981, Derwent No. 36797U-B discloses benzo[f]isoindolines. Belgium, BE 827,087, published 26 March 1974, Derwent No. 67323W/41, discloses benzo-isoindolines.

SET D (6-5-5 ring systems)

European Patent 170,093-A, published 25 july 1984, Derwent No. 86-036877/06 discloses indeno-pyrroles.

SET E (6-7-5 ring systems)

U.S. Patent 4,024,265, May 17, 1977, discloses benzo[5,6] cyclohepta[1,2-c]pyridines and their use for treating depression.

SUMMARY OF INVENTION

The present invention encompasses: 1) compounds of formula I below,

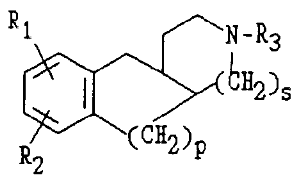

Formula I

wherein p and s are independent and may be either 1 or 2,
wherein $R_1$ is -H, -Halo, -CN, $-CO_2H$, $-CO_2R_{1-1}$, $-CONH_2$, $-CONHR_{1-1}$, $-CON(R_{1-1})_2$, -SH, - $SR_{1-1}$, $-SO_2R_{1-1}$, $-SO_2NH_2$, $-SO_2NHR_{1-1}$, $-SO_2N(R_{1-1})_2$, $-OR_{1-1}$, $-OSO_2CF_3$ $-OSO_2R_{1-1}$, $-NH_2$, $-NHR_{1-1}$, or $-N(R_{1-1})_2$; wherein $R_{1-1}$ is -H, -($C_1$-$C_8$ alkyl), -($C_1$-$C_8$ alkenyl), -($C_3$-$C_{10}$ cycloalkyl), -($C_6$ aryl), -5 or 6 member heterocyclics, -($C_1$-$C_8$ alkyl)-(5 or 6 member heterocyclics), wherein $R_2$ is -H, -Halo, -CN, $-CF_3$ -SH, or $-SR_{2-1}$; wherein $R_{2-1} = R_{1-1}$, wherein $R_3$ is -H, -($C_1$-$C_8$ alkyl), ($C_1$-$C_8$ alkenyl), -($C_6$ aryl), -($C_3$-$C_{10}$ cycloalkyl), -(5 or 6 member heterocyclics), -($C_1$-$C_8$ alkyl)-5 or 6 member heterocyclics), wherein $R_{3-1} = R_{1-1}$ or a pharmacologically acceptable salt thereof. 2. A pharmaceutical composition consisting essentially of a pharmaceutically acceptable carrier and an effective amount of a compound of formula 1. 3. A method of treating central nervous system disorders, associated with serotonin and or dopamine receptor activity comprising: administering an effective amount of a compound of formula I to a patient in need thereof.

The compounds of this invention possess selective pharmacological properties and are useful in treating central nervous system disorders including anti-depression symptoms, anxiolytic symptoms, panic attacks, obsessive-compulsive disturbances, senile dementia, emotional disturbances related to dementia disorders, and stimulation of sexual activity. Other central nervous system disorders and conditions related to central dopamine transmission such as parkinsonism, schizophrenia, and manic-depressive illness may also be treated with the compounds of this invention. The compounds of this invention are also useful to alleviate aggressive behavior, confusional delirious states and impotence. In addition to their central nervous system pharmacological activities, the compounds of this invention are also anti-diabetic, anti-obesity,antiatherosclerotic, and anti-hypertensive agents. Processes for preparation of these compounds, their pharmaceutical use and pharmaceutical preparations employing such compounds constitute further aspects of the invention.

An object of the invention is to provide compounds for therapeutic use, especially compounds having a therapeutic activity in the central nervous system. Another object is to provide compounds having an effect on the $5\text{-}HT_{1A}$ receptor in mammals including man. A further object of this invention is to provide compounds having an effect on the subclass of dopamine receptors known as the $D_2$ receptor.

The compounds of formula I where $R_3$ equals H are novel and useful as intermediates in the preparation of the compounds of formula I.

DETAILED DESCRIPTION OF THE INVENTION

The compounds of this invention are identified in two ways: by the descriptive name and reference to labeled structures contained in appropriate charts. In appropriate situations, the proper stereochemistry is also represented in the charts.

In this document the parenthetical term ($C_n$-$C_m$) is inclusive such that a compound of ($C_1$-$C_8$) would include compounds of one to 8 carbons and their isomeric forms. The various carbon moieties are defined as follows: Alkyl refers to an aliphatic hydrocarbon radical and includes branched or unbranched forms such as methyl, ethyl, n-propyl, isopropyl, n-butyl, isobutyl, sec-butyl, t-butyl, n-pentyl, isopentyl, n-hexyl, isohexyl, n-heptyl, isoheptyl, and n-octyl.

Alkoxy as represented by -$OR_1$ when $R_1$ is ($C_1$-$C_8$) alkyl refers to an alkyl radical which is attached to the remainder of the molecule by oxygen and includes branched or unbranched forms such as methoxy, ethoxy, n-propoxy, isopropoxy, n-butoxy, isobutoxy, sec-butoxy, t-butoxy, n-pentoxy, isopentoxy, n-hexoxy, isohexoxy, n-heptoxy, isoheptoxy, and n-octoxy.

Alkenyl refers to a radical of an aliphatic unsaturated hydrocarbon having at least one double bond and includes both branched and unbranched forms such as ethenyl, 1-methyl-1-ethenyl, 1-propenyl, 2-propenyl, 1-butenyl, 2-butenyl, 3-butenyl, 2-methyl- 1-butenyl, 1-pentenyl, allyl, 3-pentenyl, 4-pentenyl, 1-methyl-4-pentenyl, 3-methyl-1-pentenyl, 3-methyl-allyl, 1-hexenyl, 2-hexenyl, 3-hexenyl, 4-hexenyl, 1-methyl-4-hexenyl, 3-methyl-1-hexenyl, 3-methyl-2-hexenyl, 1-heptenyl, 2-heptenyl, 3-heptenyl, 4-heptenyl, 1-methyl-4-heptenyl, 3-methyl-1-heptenyl, 3-methyl-2 -heptenyl, 1 -octenyl, 2-octenyl, or 3-octenyl.

($C_3$-$C_{10}$)cycloalkyl refers to a radical of a saturated cyclic hydrocarbon which includes alkyl-substituted cycloalkyl, such as cyclopropyl, 2-methylcyclopropyl, 2,2-dimethylcyclopropyl, 2,3 diethylcyclopropyl, 2-butylcyclopropyl, cyclobutyl, 2-methylcyclobutyl, 3-propylcyclobutyl, cyclopentyl, 2,2-dimethylcyclopentyl, cyclohexyl, cycloheptyl, or cyclooctyl.

Examples of aryl include phenyl, naphthyl, (o-, m-, p-)tolyl, (o-, m-, p-)ethylphenyl, 2-ethyl-tolyl, 4-ethyl-o-tolyl, 5-ethyl-m-tolyl, (o-, m-, or p-)propylphenyl, 2-propyl-(o-, m-, or p-)tolyl, 4-isopropyl-2,6-xylyl, 3-propyl-4-ethylphenyl, (2,3,4-, 2,3,6-, or 2,4,5-)trimethylphenyl, (o-, m-, or p-)fluorophenyl, (o-, m-, or p-trifluoromethyl)phenyl, 4-fluoro-2,5-xylyl, (2,4-, 2,5-, 2,6-, 3,4-, or 3,5-)difluorophenyl, (o-, m-, or p-)chlorophenyl, 2-chloro-p-tolyl, (3-, 4-, 5- or 6-)chloro-o -tolyl, 4-chloro-2-propylphenyl, 2-isopropyl-4-chlorophenyl, 4-chloro-3-fluorophenyl, (3- or 4-)chloro-2-fluorophenyl, (o-, m-, or p-,)trifluorophenyl, (o-, m-, p-)ethoxyphenyl, (4- or 5-) chloro-2 -methoxy-phenyl, and 2,4-dichloro(5- or 6-)methylphenyl.

Examples of heterocyclics include: (2-, 3-, or 4-)pyridyl, imidazolyl, indolyl, $N^{in}$-formyl-indolyl, $N^{in}$-$C_2$-$C_5$alkyl-C(O)-indolyl, [1,2,4]-triazolyl, (2-, 4-, 5-)pyrimidinyl, (2-, 3-)thienyl, piperidinyl, pyrryl, pyrrolinyl, pyrrolidinyl, pyrazolyl, pyrazolinyl, pyrazolidinyl, imidazolinyl, imidazolidinyl, pyrazinyl, piperazinyl, pyridazinyl, oxazolyl, oxazolidinyl, isoxazolyl, isoxazolidinyl, morpholinyl, thiazolyl, thiazolidinyl, isothiazolyl, isothiazolidinyl, quinolinyl, isoquinolinyl, benzimidazolyl, benzothiazolyl, benzoxazolyl, furyl, thienyl, and benzothienyl. Each of these moieties may be substituted as appropriate.

Halo is halogen (fluoro, chloro, bromo or iodo) or trifluoromethyl.

LAH is lithium aluminum hydride

LDA is lithium diisopropylamide

THF is tetrahydrofuran

9-BBN-H or 9-BBN is 9-Borabicyclo[3.3.1]nonane dimer

It will be apparent to those skilled in the art that compounds of this invention may contain chiral centers. The scope of this invention includes all enantiomeric or diastereomeric forms of formula I compounds either in pure form or as mixtures of enantiomers or diastereomers. The compounds of formula I contain two asymmetric carbon atoms in the aliphatic ring moiety, including the ring carbon atoms adjacent to the nitrogen atom. The therapeutic properties of the compounds may to a greater or lesser degree depend on the stereochemistry of a particular compound.

Both organic and inorganic acids can be employed to form non-toxic pharmaceutically acceptable acid addition salts of the compounds of this invention. Illustrative acids are sulfuric, nitric, phosphoric, hydrochloric, citric, acetic, lactic, tartaric, palmoic, methanesulfonic, ethanedisulfonic, sulfamic, succinic, cyclohexylsulfamic, fumaric, maleic, and benzoic acid. These salts are readily prepared by methods known in the art.

In clinical practice the compounds of the present invention will normally be administered orally, rectally, or by injection, in the form of pharmaceutical preparations comprising the active ingredient either as a free base or as a pharmaceutically acceptable non-toxic, acid addition salt, such as the hydrochloride, lactate, acetate, mesylate, methanesulfonate, or sulfamate salt, in association with a pharmaceutically acceptable carrier. The use and administration to a patient to be treated in the clinic would be readily apparent to a physician or pharmacist of ordinary skill in the art.

In therapeutical treatment the suitable daily doses of the compounds of the invention are 1 - 2000 mg/patient for oral application, and 0.001 - 20 mg/kg for intramuscular, intravenous or subcutaneous application. The precise dosage will be apparent to an ordinarily skilled physician or pharmacologist taking into account factors such as the age, weight, sex, and medical condition of the patient being treated. Also relevant is the potency of the particular compound. The potency of a compound may be suggested by the standard tests described below.

These compounds are particularly effective anxiolytic and antidepressant agents. Central nervous system disorders and conditions related to central dopamine transmission such as parkinsonism, schizophrenia, and manic-depressive illness may also be treated with the compounds of this invention. Other uses for these compounds include panic attacks, obsessive-compulsive disturbances, and senile dementia, particularly the emotional disturbances seen in dementia disorders. In addition, central 5-HT receptor activation are believed to be involved in mediating sexual disorder. These compounds would be useful to stimulate sexual activity and to alleviate impotence. The compounds of this invention are also useful to alleviate aggressive behavior and confusional delirious states.

The compounds of this invention can be made in accordance with the processes illustrated in CHARTS A, B and C. The reactions and properties are divided into two parts. PART I, immediately below, contains compounds related to CHARTS A and B. The reactions of compounds of CHARTS A and B are followed by the biological data for the compounds of CHARTS A and B followed by detailed preparation steps. The compound claims that correspond to PART I, the compounds of CHARTS A and B are claims 14 - 24. Part II contains the COMPOUNDS that correspond to CHART C, the reactions, the corresponding biological data and the detailed preparations. The compound claims that correspond to PART II are claims 2 - 13.

PART I

Reactions of CHART A

Step 1, the starting ketone, A1, is refluxed with the sodium salt of triethyl phosphonoacetate to give the ester, $A_2$, along with some of its exo double bond isomer. The undesired isomer is removed by treating the isomeric mixture with LDA followed by quenching with acetic acid. Step 2, the ester, A2, is reduced to the alcohol, A3, with LAH. Step 3, the alcohol, A3, is treated with silylchloride to protect the alcohol as the *t*-butyldimethylsilyl ether A4.

Step 4, the silyl ether, A4, is refluxed with 9-BBN-H in THF and then treated with a mixture of bromo ethylacetate and potassium 2,6-di-*t*-butylphenoxide to give the ester, A5. Step 5, the silyl group is removed with aqueous acid to afford the alcohol, A6. Step 6, A6 is reduced with lithium borohydride to afford the diol, A7. Step 7, the diol is converted into the dibromide, A8, with NBS and triphenylphosphine. Step 8, the dibromide is heated with optically pure $\alpha$-methylbenzylamine to afford a mixture of diastereomeric azepines, A9, which are separated by chromatography. The diastereomers are separately carried on to the final optically pure enantiomers using the following procedures. Step 9, optically active azepine, A9, is treated with *t*-butyllithium followed by trimethylsilylisocyanate to afford the carboxamido azepine, A10. Step 10, hydrogenation over palladium hydroxide afforded A11. Step 11, the hydrogenated azepine was alkylated with bromopropane to afford A12. Step 12, the carboxamido group is replaced with a cyano group, A13.

Structures are shown in CHART A.

Reactions of CHART B

Step 1, indanone, B1, is treated with pyrrolidine to convert it into the eneamine, B2. Step 2, the eneamine is alkylated with ethyl bromoacetate to afford the carboxylate, B3. Step 3, the carboxylate undergoes a Reformatsky reaction with ethyl bromoacetate to afford the diester, B4. Step 4, the diester is reduced with triethylsilane in trifluoroacetic acid to obtain the reduced diester, B5. Step 5, the reduced diester is further reduced with LAH to afford the diol, B6. Step 6, the diol is converted into the di-*p*-toluenesulfonate, A7. Step 7, the di-p-toluenesulfonate is converted into the azepines B8 and B9 (trans and cis- fused rings, respectively).

Structures are shown in CHART B.

The compounds of this invention have high oral potency and a long duration of action. This exceptionally good bioavailability combined with a long period of activity are beneficial to effective clinical treatment. The preferred compounds of PART I are not only active but have increased bioavailability as shown by metabolism studies. See PART I - TABLES I-IV, below. One preferred compound is (-)-trans-10-carboxamido-5a,10b-dihydro-3N-n-propyl-6H-indeno[1,2-d]azepine, compound number 3, below.

The compounds of this invention are useful both as intermediates to produce other compounds and they

are useful to treat central nervous system disorders. The utility of the compounds of this invention to treat central nervous system disorders is shown in behavioral, physiological and biochemical tests. These tests are described below.

CNS Receptor Binding Assay (PART I - TABLE I): The Central Nervous System Binding Assay measures the percent inhibibition from experiments employing test compounds at 1 microMolar concentration competing with various radioligands for binding to whole brain membranes, membranes prepared from specific brain structures, or membranes prepared from cell lines expressing cloned receptors. When percent inhibition is equal to 100 the test compound binds as well as the standard compound. The standard compound for the 5-$HT_{1A}$ receptor is 8-OH-DPAT. The standard compound for the dopamine D1 receptor is SCH 23390.

5-HT and DA Cell Firing (PART I - TABLE II): Glass microelectrodes filled with pontamine sky blue in 2M NaCl were used for extracellular recordings from Sprague-Dawley rats anesthetized with chloral hydrate (400 mg/kg i.p.). Drugs were injected intravenous and five neurons were located in the dorsal raphe nucleus according to Aghajanian et al., J. Pharmacol. Expo Ther. 137:178 (1970). DA neurons were located in the substantia nigra pars compacta (SNPC) and identified according to Bunney et al., J. Pharmacol. Exp. Ther. 185:560 (1973). For 5-$HT_{1A}$ agonist effects, the ED50 is the dose required to depress dorsal raphe neuron firing by 50% of the maximal depression obtainable. Lum and Piercey, Eur. J. Pharmacol. 149:9-15 (1988). For DA agonist effects, the ED50 is the dose required to depress SNPC neuron firing by 50% for full agonists, or for partial agonists, the dose required to depress SNPC neuron firing by 50% of the maximal depression attainable. Piercey and Hoffmann, J. Pharmacol. Exp. Ther. 243:391, (1987). For DA antagonists, the ED50 is the dose required to reverse the depression of SNPC neuron firing caused by a DA agonist (usually either 1-3 mg/kg amphetamine, or 100 ug/kg apomorphine). For partial DA agonists, the antagonist ED50 is the dose required to reverse the agonist-induced depression of SNPC neuron firing by 50% of the maximum reversal. Piercey and Hoffmann, J. Pharmacol. Exp. Ther., 243:391 (1987).

Sympathetic Nerve Discharge (SND) (PART I - TABLE III): SND ED50 (mp/kp): The i.v. mg/kg dose causing a 50% depression in SND in chloralose anesthetized cats. Max. Decr. SND % Control: The maximum inhibition of sympathetic activity observed in the dose range tested (0.001-1.0 mg/kg i.v.). %BP at SND ED50: The blood pressure of the chloralose anesthetized cats in percent control at the dose causing 50% depression in SND. Max Decr. BP% Control: The maximum reduction in blood pressure as percent of the control blood pressure in the same animals observed in the dose range tested (0.001-1.0 mg/kg i.v.).

Metabolism (PART I - TABLE IV): Intrinsic clearance of 3 concentrations of compound (2,5 and 15 ug/ml) following a 60 minute incubation at 37°C in the presence of a suspension of freshly prepared rat hepatocytes (5.0 million cells/ml). Aliquots of each incubate were withdrawn during the incubation and analyzed for parent compound using HPLC methodology. Intrinsic clearance is expressed as ml/min/5 million cells. Metabolic stability relative to the control, (±) 1-formyl-6,7,8,9-tetrahydro-N-di-n-propyl-8-amino-3H-benz[e]indole, is determined.

Hypothermia (PART I - TABLE IV): Starting with a dose of 30 mg/kg, four mice are injected subcutaneously with test compound. Twenty minutes later, the number of animals whose body temperature has decreased by 2°C or more are counted. If all four animals reach criteria, the drug is considered "active," and subsequent readings are taken at 60 and 120 minutes after administration of the drug. The time for the last statistically significant drug effect on mean body temperature is indicated in minutes. For all "active" compounds, doses are lowered by 0.5 log intervals until a dose which does not lower body temperature by 2°C in any animal is found. Potency is given as mg/kg ED50 (dose required to depress temperature in two of four mice) as measured by Spearman-Karber statistics.

The compounds below have been subjected to one or more of the biological tests described above. PART I - TABLES I - IV use the arbitrary numbers assigned below.

| Compound Number | Name |
|---|---|
| 1 | **cis-7-chloro-10-methoxy-5a,10b-dihydro-3N-*n*-propyl-6H-indeno[1,2-d]azepine,** see CHART B structure B9. |
| 2 | ***trans*-7-chloro-10-methoxy-5a,10b-dihydro-3N-*n*-propyl-6H-indeno[1,2-d]azepine,** see CHART B structure B9. |
| 3 | **(-)-*trans*-10-carboxamido-5a,10b-dihydro-3N-*n*-propyl-6H-indeno[1,2-d]azepine,** see CHART A structure A12. |
| 4 | **(+)-*trans*-10-carboxamido-5a,10b-dihydro-3N-*n*-propyl-6H-ideno[1,2-d]azepine,** see CHART A structure A12. |
| 5 | **(-)-*trans*-7-carboxamido-5a,10b-dihydro-3N-*n*-propyl-6H-indeno[1,2-d]azepine,** see CHART A structure A12. |
| 6 | **(+)-*trans*-7-carboxamido-5a,10b-dihydro-3N-*n*-propyl-6H-indeno[1,2-d]azepine,** see CHART A structure A12. |
| 7 | **(-)-*trans*-7-cyano-5a,10b-dihydro-3N-*n*-propyl-6H-indeno[1,2-d]azepine,** see CHART A structure A13. |
| 8 | **(+)-*trans*-7-cyano-5a,10b-dihydro-3N-*n*-propyl-6H-indeno[1,2-d]azepine,** see CHART A structure A13. |

PART I - TABLE I

BIOLOGICAL DATA - CNS Receptor Binding

| Compound No. | $5\text{-}HT_{1A}$ % inhibition | Dopamine - D1 % inhibition | Dopamine - D2 % inhibition |
|---|---|---|---|
| 1 | 99 | 57 | 74 |
| 2 | -- | -- | -- |
| 3 | 93 | 13 | 45 |
| 4 | 21 | 0 | 2 |
| 5 | 24 | 10 | 24 |
| 6 | 18 | 50 | 31 |
| 7 | 17 | 11 | 22 |
| 8 | 15 | 7 | 35 |

PART I - TABLE II

BIOLOGICAL DATA - 5HT and DA Cell Firing

| Compound No. | $5\text{-}HT_{1A}$ Firing (mg/kg) | DA Firing (mg/kg) |
|---|---|---|
| 2 | -- | 0.065 |

PART I - TABLE III
BIOLOGICAL DATA - Sympathetic Nerve Discharge (SND)

| Compound No. | SND ED50 (mg/kg) | Max. Decr. SND % control | %BP at SND ED50 | Max. Decr. BP BP % control |
|---|---|---|---|---|
| 3 | 1.0 | 1.0 | 66.0 | 66.0 |

PART I - TABLE IV
BIOLOGICAL DATA - Metabolism and Hypothermia

| Compound No. | Metabolic Stability (control is 1.0) | Hypothermia (mg/kg) | Duration (minutes) |
|---|---|---|---|
| 2 | -- | 13 | 120 |
| 3 | 0.95 | 0.97 (subcutaneous) | -- |
| 3 | | 1.3 (oral) | -- |
| 4 | 0.39 | -- | -- |

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

PREPARATIONS AND EXAMPLES FROM CHART A

STEP 1

Preparation A1(a): **7-bromo-4-chloro-1-carboethoxymethyl-3H-indene**, see CHART A, STEP 1, compound A2.

Triethyl phosphonoacetate (34.2 ml) and sodium hydride (7.92 g of 50 % slurry in oil) are placed sequentially in a lask with THF (300 ml). After hydrogen evolution ceases, the solution is cooled to 0°C and a THF (50 ml) solution of 7-bromo-4-chloroindanone is added. The solution is brought to reflux and kept there for 18 hours. The solution is then cooled and partitioned into ether and water. The ether layer is washed with water (2X) and dried over anhydrous sodium sulfate and the solvent removed *in vacuo*. NMR shows a 3:1 ratio of the endo olefin to the exo. This mixture is added to a THF solution of lithium diisopropylamide (1 eq.) at -78°C. After five minutes acetic acid (1 eq.) is added in THF (10 ml) and the solution is allowed to warm to 25°C where it is partitioned between water and ether. The ether layer is washed with 2N aqueous hydrochloric acid followed by water, saturated aqueous sodium bicarbonate, and brine. The ether solution is dried over anhydrous sodium sulfate and the solvent removed *in vacuo* to afford 31 g of the title compound after chromatography with ethyl acetate/hexane (2:98).

Preparation A1(b): **4-bromo-1-carboethoxymethyl-3H-indene**, see CHART A, STEP 1, compound A2.

Use preparation A1(a), only start with 4-bromo-1-indanone.

STEP 2

Preparation A2(a), **7-bromo-4-chloro-1-(hydroxyeth-2-yl)-3H-indene**, see CHART A, STEP 2, compound A3.

A solution of 7-bromo-4-chloro-1-carboethoxymethyl-3H-indene (25.61 g, 81.2 mmol) in diethylether (150 ml) is added over a 10 minute period to a suspension of lithium aluminum hydride (10.0 g, 0.263 mol) in diethylether (250 ml) with stirring at 0°C. The cold bath is removed, and the mixture is stirred for 15 minutes. The mixture again is cooled to 0°C, and water (10 ml), 15% NaOH (10 ml), and water (30 ml) are added in succession. The cold bath is removed, and the mixture is stirred for 10 minutes. The mixture is filtered, and the precipitate is washed well with diethylether. The filtrate is dried ($MgSO_4$), and the solvent is removed under vacuum to leave the title compound as a yellow solid (21.67 g, 98%).

Preparation A2(b): **4-bromo-1-(hydroxyeth-2-yl)-3H-indene**, see CHART A, STEP 2, compound A3.

Use preparation A2(a), only start with 4-bromo-1-carboethoxymethyl-3H-indene.

STEP 3

Preparation A3(a): **1-(*t*-butyldimethysilyloxyeth-2-yl)-7-bromo-4-chloro-3H-indene**, see CHART A, STEP 3, compound A4.

In this procedure 7-Bromo-4-chloro-1-(hydroxyeth-2-yl)-3H-indene (28.23g,103.2 mmol) and imidazole (15.4 g, 0.226 mol) are dissolved in dimethylformamide (200 ml), cooled to 0°C, and t-butyldimethylsilyl chloride (17.08 g, 0.113 mol) is added. The mixture is stirred at room temperature for 12 hours, and partitioned between diethylether and water. The ether solution is washed several times with water and once with brine. The solution is dried ($Na_2SO_4$), and the solvent is removed under vacuum to leave an amber oil (39.94 g). Purification by flash chromatography (230-400 mesh silica gel; 10% toluene in hexane) gives the title compound as an amber oil (37.26 g).

Preparation A3(b): **1-(*t*-butyldimethylsilyloxyeth-2-yl)-4-bromo-3H-indene**, see CHART A, STEP 3, compound A4.

Use Preparation A3(a) only start with 4-bromo-1-(hydroxyeth-2-yl)-3H-indene.

STEP 4

Preparation A4(a): ***trans*-7-bromo-1-(*t*-butyldimethylsilyloxyeth-2-yl)-2-carboethoxymethyl-4-chloro-2,3-dihydro-3H-indene**, see CHART A, STEP 4, compound A5.

Add 9-Borabicyclo[3.3.1]nonane (9-BBN-H) dimer (11.0 g, 0.045 mol, 1.05 eq.) to a solution of 1 -(*t*-butyldimethylsilyloxyeth-2-yl)-7-bromo-4-chloro-3H-indene (33.28 g, 85.8 mmol) in dry tetrahydrofuran. Heat the mixture at reflux under argon in an oil bath maintained at 100°C for 41 hours and cooled in ice. A solution of 2,6-di-t-butylphenol (23.02 g, 0. 112 mol) in dry tetrahydrofuran (200 ml) is cooled to 0°C, and a solution of potassium t-butoxide in tetrahydrofuran (1.0 M, 90.1 ml, 90.1 mmol) is added over 10 minutes. The mixture is stirred at room temperature for 15 minutes and again cooled in ice. The solution is added to the borane solution above via needlestock. Ethyl bromoacetate (15.1 g, 90.2 mmol) is added dropwise, and the mixture is stirred at 0°C for 30 minutes, room temperature for 2 hours, and at reflux for 15 minutes. The mixture is diluted with diethylether and washed with water, 10% sodium carbonate solution, and brine. The solution is dried ($MgSO_4$), and the solvent is removed under vacuum to leave an oil. Purification by flash chromatography (230-400 mesh silica gel; pure hexane to 2% ethyl acetate in hexane) gives the title compound as an oil (30.2 g, 74% yield).

Preparation A4(b): ***trans*-4-bromo-1-(*t*-butyldimethylsilyloxyeth-2-yl)-2-carboethoxymethyl-2,3-dihydro-3H-indene,** see CHART A, STEP 4, compound A5.

Use procedure A4(a) with the following modifications. 1 -(*t*-Butyldimethylsilyloxyeth-2-yl)4-bromo-3H-indene (38.8 g, 109.9 mmol) and 9-BBN-H (dimer) (14 g, 115.4 mmol) are dissolved in THF (100 ml) under a nitrogen atmosphere. This solution is refluxed for 48 hours and then cooled. A slurry of potassium 2,6-di-*t*-butylphenoxide in THF is prepared by dissolving 2,6-di-*t*-butylphenol (29.5 g) in THF (50 ml) and adding a 1.0 M THF solution of potassium *t*-butoxide (115.4 ml). The slurry is stirred for 10 minutes and then cooled to 0°C. The solution of the organoborane is then added. Ethyl bromoacetate (12.8 ml) is added over a 3 minute period. The slurry is stirred at 0°C for 1 hour and then allowed to stir at 25°C for 1 hour. The flask is cooled to 0°C and quenched with water and then partitioned between water and ether. The ether layer is washed with water (2X) and brine, then dried over sodium sulfate and the solvent removed *in vacuo*. The resulting oil is placed on flash silica gel (6 cm X 45 cm) and eluted with ether/hexane (0.5:99.5). This is raised to 5:95 to elute off the product which is obtained as an oil (18.7 g, 38 % yield).

STEP 5

Preparation A5(a): ***trans*-7-bromo-2-carboethoxymethyl-4-chloro-2,3-dihydro-1-(hydroxyeth-2-yl)-3H-indene,** see CHART A, STEP 5, compound A6.

Acetic acid (180 ml) is added to a solution of *trans*-7-bromo-1-(*t*-butyldimethylsilyloxyeth-2-yl)-2-carboethoxymethyl-4-chloro-2,3-dihydro-3H-indene (29.2 g, 61.3 mmol) in tetrahydrofuran (60 ml). Water (60 ml) is added, and the mixture is stirred at room temperature for 18 hours. The solvent is removed under vacuum, and the oil is dissolved in diethylether, washed twice with 10% aqueous sodium carbonate followed by brine, and the solution is dried over magnesium sulfate. The solvent is removed under vacuum leaves a yellow oil (24.5 g). Purification by flash chromatography (230-400 mesh silica gel, 20% ethyl acetate in hexane) gives the title compound as a colorless oil (18.4 g, 60% yield from the olefin).

Preparation A5(b): ***trans*-4-bromo-2-carboethoxymethyl-2,3-dihydro-1-(hydroxyeth-2-yl)-3H-indene**, see CHART A, STEP 5, compound A7.

Use preparation A5(a) with the following modifications. *Trans*-4-bromo-1-(*t*-butyldimethylsilyloxyeth -2-yl)-2-carboethoxymethyl-2,3-dihydro-3H-indene (46 g) is dissolved in acetic acid/THF/water (3:1:1, 250 ml) and stirred for 16 hours. The solvent is removed *in vacuo* to obtain 22.3 g of pure oil.

STEP 6

Preparation A6(a): ***trans*-7-bromo-4- chloro-2,3-dihydro-1,2-di(hydroxyeth-2-yl)-3H-indene,** see CHART A, STEP 6, compound A7.

Lithium borohydride (2.0 M in tetrahydrofuran, 45 ml, 90 mmol) is added to a solution of*trans*-7-bromo-2-carboethoxymethyl-4-chloro-2,3-dihydro-1-(hydroxyeth-2-yl)-3H-indene(16.07 g, 0.0444 mol) in diethylether (400 ml) at room temperature. The mixture is stirred for 3 hours, allowed to stand at 0°C for 15.5 hours, and refluxed for 30 minutes. The mixture is cooled in ice, and water (100 ml) is slowly added. When the reaction subsided, 10% hydrochloric acid (100 ml) is slowly added, and the mixture is stirred for 30 minutes. The layers are separated, and the aqueous layer is extracted with diethylether. The combined ether solution is washed with water, saturated aqueous sodium bicarbonate, and brine. The solution is dried over magnesium sulfate, and the solvent removed under vacuum to leave the title compound as a colorless oil (15.0 g,100%).

Preparation A6(b): ***trans*-4-bromo-2,3-dihydro-1,2-di(hydroxyeth-2-yl)-3H-indene**, see CHART A, STEP 6, compound A7.

Use preparation A6(a) only start with *trans*-4-bromo-2-carboethoxymethyl-2,3-dihydro-1-hydroxyeth-2-yl)-3H-indene.

STEP 7

Preparation A7(a): ***trans*-7-bromo-4-chloro-2,3-dihydro-1,2-di(bromoeth-2-yl)3H-indene,** see CHART A, STEP 7, compound A8.

In this procedure, *trans*-7-bromo-4-chloro-2,3-dihydro-1,2-di(hydroxyeth-2-yl)-3H-indene (19.7 g) is dissolved in methylene chloride (300 ml) and THF (500 ml) with triphenylphosphine (38 g) and cooled to 0°C. N-Bromosuccinimide (25 g) is added in portions. The solution is stirred for 20 minutes and the solvent removed *in vacuo*. The residue is dissolved in ethyl acetate (200 ml) and silica gel (300 g) is added. With swirling, hexane

(800 ml) is added. The slurry is filtered and the solvent removed *in vacuo*. The residue is eluted down a flash silica gel column (4 cm X 25 cm) with ethyl acetate/hexane (10:90). Solvent removal *in vacuo* afforded the dibromide in 89 % yield.

Preparation A7(b): ***trans*-4-bromo-1,2-di(bromoeth-2-yl)-2,3-dihydro-3H-indene,** see CHART A, STEP 7, compound A8.

Use preparation A7(a) only start with *trans*-4-bromo-2,3-dihydro-1,2-di(hydroxyeth-2-yl)3H-indene (19.7 g, 69.1 mmol). Title compound is afforded in 89% yield (25.3 g of an oil).

STEP 8

Preparation A8(a): preparation and separation of the diastereomers of A9, ***trans*-10-bromo-7-chloro -3N-[(S)-methylbenzyl]-6H-5a,10b-dihydroindeno[1,2-d]azepine**, see CHART A, STEP 8, compound A9.

A mixture of *trans*-7-bromo-4-chloro-2,3-dihydro-1,2-di(bromoeth-2-yl)-3H-indene (19.31 g, 0.0307 mol), (S)-(-)-α-methylbenzylamine (3.91 g, 0.0323 mol), and potassium carbonate (12.73 g, 0.0921 mol) in acetonitrile (200 ml) is stirred at reflux on the steambath for 48 hours. The solvent is removed under vacuum, and the residue is partitioned between water and diethylether. The aqueous layer is extracted with diethylether, and the combined organics are washed with brine and dried ($MgSO_4$). The solvent is removed under vacuum to leave an orange oil (14.22 g). Purification by flash chromatography (230-400 mesh silica gel, 5-30 % ethyl acetate in hexane) give the desired amine as a mixture of diastereomers (7.05 g). Further purification by medium pressure liquid chromatography in a Michell-Miller column (230-400 mesh silica gel, 2-2.5% ethyl acetate in hexane) gives two diastereomers. Diastereomer 1 (higher Rf)(2.88 g). Diastereomer 2 (lower Rf) (2.52 g).

Preparation A8(b): preparation and separation ofthe diastereomers of A9, ***trans*-7-bromo-3N -[(S)-methylbenzyl]-6H-5a,10b-dihydroindeno[1,2-d]azepine,** see CHART A, STEP 8, compound A9.

Use procedure A8(a) only start with *trans*-4-bromo-1,2-di(bromoeth-2-yl)-2,3-dihydro-3H-indene (25.3 g). The reaction is refluxed for 48 hours to afford compound. The diastereomers are separated with flash silica gel chromatography (5cm X 30cm) eluting with ether/hexane (1:99). The higher Rf fraction is isolated as a crystalline solid (m.p. 117°) by crystallizing from hexane/toluene (7.2 g) $[\alpha]_{25}^{589}$ = -8.45° (c = 0.355, chloroform). The lower Rf diastereomer remains an oil (8.75 g). $[\alpha]_{25}^{589}$ = +5.82° (c = 1.545, chloroform). The remainder is a mixture of starting material and diastereomeric products which are resubjected to the reaction conditions.

STEP 9

Preparation A9(a)(1-2): preparation of two diastereomers of A10, see CHART A, STEP 9, compound A10, follow the appropriate paragraph below.

Preparation A9(a)(1): preparation of ***trans*-10-carboxamido-7-chloro-5a,10b-dihydro-3N-[(S)-methylbenzyl]-6H-indeno[1,2-d]azepine** from diastereomer 1. A solution of *trans*-10-bromo-7 -chloro-3N-[(S)-methylbenzyl]-6H-5a,10b-dihydroindeno[1,2-d]azepine (diastereomer 1; 2.88 g, 7.12 mmol) in dry, argon degassed tetrahydrofuran (30 ml) is cooled to -78°C, and *t*-butyllithium (1.7 M in pentane, 8.4 ml, 14.3 mmol) is added over 15 seconds. The mixture is stirred at -78°C for 5 minutes and added via needle stock to a solution of trimethylsilylisocyanate (2.42 g of 85 %, 17.9 mmol) in dry tetrahydrofuran (10 ml) and dioxane (15 ml) at -78°C. The cold bath is removed and the mixture is allowed to warm to room temperature. Water is added, the mixture is stirred for 15 minutes, and the volatiles were removed under vacuum. The residue is shaken with diethylether and water, the precipitate is filtered, washed with diethylether, and dried under vacuum at 60°C to give a solid (1.86 g). A sample (0.20 g) is crystallized from ethanol to give the title compound as a colorless solid (0.15 g, m.p. 231-232°C).

Preparation A9(a)(2): preparation of ***trans*-10-carboxamido-7-chloro-5a,10b-dihydro-3N-[(S)-methylbenzyl]-6H-indeno[1,2-d]azepine** from diastereomer 2, see CHART A, STEP 9, compound A10.

The amide is prepared in a manner similar to that for diastereomer 1, using procedure A9(a)(1), except starting with diastereomer 2 of *trans*-10-bromo-7-chloro-3N-[(S)-methylbenzyl]-6H-5a,10b-dihydroindeno[1,2-d]azepine. The product is a colorless solid. A sample may be crystallized from acetonitrile (m.p. 192-193°C).

Preparation A9(b)(1-2): preparation of two diastereomers of A10, see CHART A, STEP 9, compound A10, follow the appropriate paragraph below.

Preparation A9(b)(1), preparation of (+)-***trans*-7-carboxamido-5a,10b-dihydro-3N-[(S)-methylbenzyl]-6H-indeno[1,2-d]azepine.**

Use preparation A9(a)(1) only start with (+)-*trans*-7-bromo-3N-[(S)-methylbenzyl]-6H-5a, 10b-dihydroindeno[1,2-d]azepine (7.26 g). This produces 5.57 g of the isomer as solid. $[\alpha]_{25}^{589}$ = +29.11° (c = 0.56 methanol).

Preparation A9(b)(2): **(-)-*trans*-7-carboxamido-5a,10b-dihydro-3N-[(S)-methylbenzyl]6H-indeno[1,2-d]azepine.**

Use preparation A9(a)(1) only start with (-)-*trans*-7-bromo-3N-[(S)-methylbenzyl]-6H-5a, 10b-dihydroindeno[1,2-d]azepine (8.75 g). Afforded 6.7 g of the title compound as a solid. $[\alpha]_{25}^{589}$ = -25.97° (c = 1.525 methanol).

Preparation A9(c): ***trans*-10-aminosulfonyl-7-chloro-5a,10b-dihydro-3N-[(S)methylbenzyl]-6H-indeno[1,2-d]azepine**, CHART A, STEP 9, compound A10.

The diastereomeric title sulfonamide is prepared from the diastereomer of *trans*-10-bromo-7 -chloro-3N-[(S)-methylbenzyl]-6H-5a, 10b-dihydroindeno[1,2-d]azepine, see procedure A8(a), by performing a metal-halogen exchange with *t*-butyllithium, as in procedure A9(a)(1), followed by conversion of the aryllithium to the sulfonamide according to the reference: S. L. Graham, et al., J. Med. Chem., 32:2548 (1989).

STEP 10

Preparation A10(a)(1-2), Examples 1-2: preparation of two diastereomers of A11, see CHART A, STEP 10, compound A11, follow the appropriate paragraph below.

Preparation A10(a)(1), Example 1: **(+)-*trans*-1-carboxamido-5a,10b-dihydro-6H-indeno[1,2-d]azepine.**

A mixture of (+)-*trans*-10-carboxamido-5a, 10b-dihydro-3N-[(S)-methylbenzyl]-6H-indeno [1,2-d]azepine (1.70 g, 4.61 mmol), 20% palladium hydroxide on carbon (0.65 g), 12 N hydrochloric acid (0.4 ml) in water (15 ml), and absolute ethanol (75 ml) is hydrogenated in a Parr apparatus for 16 hours with an initial hydrogen pressure of 50 psi. The mixture is filtered through diatomaceous earth and washed well with ethanol. The combined filtrate is evaporated leaving a foam (1.37 g).

Procedure A10(a)(2), Example 2: **(-)-*trans*-10-carboxamido-5a,10b-dihydro-6H-indeno-[1,2-d]azepine.**

Use the same procedure as A10(a)(1), except substituting (-)-*trans*- 10-carboxamido-5a, 10b-dihydro-3N-[(S)-methylbenzyl]-6H-indeno[1,2-d]azepine in place of the equivalent (+) isomer.

Preparation A10(b), Examples 3-4, preparation of two diastereomers of A11, see CHART A, STEP 10, compound A11, from the appropriate paragraph below.

Preparation A10(b)(1), Example 3: **(+)-*trans*-7-carboxamido-5a,10b-dihydro-6H-indeno[1,2-d]azepine.**

Use procedure A10(a)(1) only start with (+)-*trans*-7-carboxamido-5a,10b-dihydro-3N-[(S)methylbenzyl]-6H-indeno[1,2-d]azepine (5.5 g). The isomer is produced as a white solid, m.p. 222°C decomp. $[\alpha]_{25}^{589}$ = + 28.68° (c = 0.68 methanol).

Preparation A10(b)(2), Example 4: **(-)-*trans*-7-carboxamido-5a,10b-dihydro-6H-indeno[1,2-d]azepine.**

Use preparation A10(a)(1) only start with (-)-*trans*-7-carboxamido-5a,10b-dihydro-3N-[(S)methylbenzyl]-6H-indeno[1,2-d]azepine. The title compound is produced as a white solid, m.p. 223°C,decomp. $[\alpha]_{25}^{589}$ = -29.43° (c = 0.72 methanol).

Preparation A10(c): ***trans*-10-aminosulfonyl-5a,10b-dihydro-6H-indeno[1,2-d]azepine**, see CHART A, STEP 10, compound A11.

Use procedure 10(a)(1) only start with ***trans*-10-aminosulfonyl-7-chloro-5a,10b-dihydro-3N -[(S)-methylbenzyl]-6H-indeno[1,2-d]azepine,** from preparation 9(c).

STEP 11

Preparation A11(a)(1-2), Examples 5-6: preparation of two diastereomers of A12, see CHART A, STEP 11, compound A12, follow the appropriate paragraph below.

Preparation A11(a)(1), Example 5: **(+)-*trans*-10-carboxamido-5a,10b-dihydro-3N-*n*-propyl-6H-indeno[1,2-d]azepine.**

A mixture of (+)-*trans*-10-carboxamido-5a,10b-dihydro-6H-indeno[1,2-d]azepine hydrochloride (1.37 g, 4.61 mmol), 1-bromopropane (1.76 g, 14.3 mmol), potassium carbonate (1.91 g, 13.8 mmol), triethylamine (0.73 g, 7.2 mmol), and acetonitrile (50 ml) is stirred at reflux for 17 hours. The solvent is removed under vacuum, and the residue is partitioned between water and 1:1 THF/diethylether. The aqueous layer is extracted again with the same solvent, and the combined extracts were washed with brine and dried ($MgSO_4$). The solvent is removed under vacuum to leave a solid (1.04 g). Crystallization from acetonitrile gives colorless crystals (m.p. 198-199°C). $[\alpha]_D$ = + 218.7° (c = 0.785, 25°C, THF).

Preparation A11(a)(2), Example 6: **(-)-*trans*-10-carboxamido-5a,10b-dihydro-3N-*n*-propyl-6H-indeno[1,2-d]azepine.**

Use the same preparation as above except substituting (-)-*trans*-10-carboxamido-5a, 10b-dihydro -6H-indeno[1,2-d]azepine hydrochloride for the equivalent (+) isomer (0.85 g, m.p. 198-199°C). $[\alpha]_D$ = -222.3° (c = 0.865, 25°C, THF).

Preparation A11(b)(1-2), Examples 7-8: preparation of two diastereomers of A12, see CHART A, STEP 11, compound A12, from the appropriate paragraph below.

Preparation A11(b)(1), Example 7: **(+)-*trans*-7-carboxamido-5a,10b-dihydro-3N-*n*-propyl-6H-indeno[1,2-d]azepine.**

Use preparation A11(a) only start with (+)-*trans*-7-carboxamido-5a,10b-dihydro-6H-indeno [1,2-d]azepine. The isomer is isolated as a white solid which is converted into the fumaric acid salt with one equal of acid in methanol/ether (m.p. 185°C). $[\alpha]_{25}^{589}$ = +26.47° (c = 0.665 methanol).

Preparation A11(b)(2), Example 8: **(-)-*trans*-7-carboxamido-5a,10b-dihydro-3N-*n*-propyl-6H-indeno[1,2-d]azepine.**

Use preparation A11 (a) only start with (-)-*trans*-7-carboxamido-5a,10b-dihydro-6H-indeno [1,2-d]azepine. The isomer is isolated as a white solid which is converted into the fumaric acid salt with one equal of acid in methanol/ether (m.p. 185°). $[\alpha]_{25}^{589}$ = -23.84° (c = 0.99 methanol).

Preparation A11(c): ***trans*-10-aminosulfonyl-5a,10b-dihydro-3N-n-propyl-6H-indeno[1,2-d]azepine,** see CHART A, STEP 10, compound A11.

Use procedure A11 (a) only start with *trans*-10-aminosulfonyl-5a,10b-dihydro-6H-indeno [1,2-d]azepine, from procedure A10(c).

STEP 12

Preparation A12(a)(1-2): preparation of two diastereomers of A13, see CHART A, STEP 12, compound A13, follow the appropriate paragraph below.

Preparation A12(a)(1): **(+)-*trans*-10-cyano-5a,10b-dihydro-3N-*n*-propyl-6H-indeno[1,2-d]azepine.** (+)-*trans*-10-carboxamido-5a,10b-dihydro-3n-*n*-propyl-6H-indeno[1,2-d]azepine(0.33 g, 1.22 mmol) is dissolved in THF (15 mL) and triethylamine (1.0 mL, 7.42 mmol) then cooled to 0°C. Titanium (IV) Chloride (0.33 mL,

3.04 mmol) is added. After stirring at 20°C for 2 hours, it is partitioned between methylene chloride and dilute aqueous sodium carbonate. The organic layer is washed with water and brine, then dried over sodium sulfate. Product is dissolved in ether and hydrochloric acid in ether is added. Product is recrystallized from methanol and ether.

Preparation A 12(a)(2): **(-)-*trans*-10-cyano-5a,10b-dihydro-3N-*n*-propyl-6H-indeno[1,2-d]azepine.** (-)-*trans*-10-carboxamido-5a,10b-dihydro-3n-*n*-propyl-6H-indeno[1,2-d]azepine (0.28 g, 1.02 mmol) is dissolved in THF (15 mL) and triethylamine (0.87 mL, 6.24 mmol) then cooled to 0°C. Titanium (IV) Chloride (0.28 mL, 2.56 mmol) is added. After stirring at 20°C for 2 hours, it is partitioned between methylene chloride and dilute aqueous sodium carbonate. The organic layer is washed with water and brine. The product is dissolved in ether and hydrochloric acid in ether is added. Product is recrystallized from methanol and ether.

Preparation A12(b)(1-2), Examples 9-10: preparation of two diastereomers of A13, see CHART A, STEP 12, compound A 13, follow the appropriate paragraph below.

Preparation A12(b)(1), Example 9: **(+)-*trans*-7-cyano-5a,10b-dihydro-3N-*n*-propyl-6H-indeno[1,2-d]azepine.**

The title compound is prepared from (+)-*trans*-7-carboxamido-5a,10b-dihydro-3n-*n*-propyl-6H -indeno[1,2-d]azepine (0.33 g, 1.22 mmol) is dissolved in THF (15 mL) and triethylamine (1.0 mL, 7.42 mmol) then cooled to 0°C. Titanium (IV) Chloride (0.33 mL, 3.04 mmol) is added. After stirring at 20°C for 2 hours, it is partitioned between methylene chloride and dilute aqueous sodium carbonate. The organic layer is washed with water and brine, then dried over sodium sulfate to yield brown oil (0.29 g). The oil is dissolved in ether and hydrochloric acid in ether is added. The tan solid is recrystallized from methanol and ether to give a gray solid (0.22 g, m.p. 252°C).

Preparation A12(b)(2), Example 10: **(-)-*trans*-7-cyano-5a,10b-dihydro-3N-*n*-propyl-6H-indeno [1,2-d]azepine.**

The title compound is prepared from (-)-*trans*-7-carboxamido-5a,10b-dihydro-3n-*n*-propyl-6H-indeno[1,2-d]azepine (0.28 g, 1.02 mmol) is dissolved in THF (15 mL) and triethylamine (0.87 mL, 6.24 mmol) then cooled to 0°C. Titanium (IV) Chloride (0.28 mL, 2.56 mmol) is added. After stirring at 20°C for 2 hours, it is partitioned between methylene chloride and dilute aqueous sodium carbonate. The organic layer is washed with water and brine, then dried over sodium sulfate to yield brown oil (0.23 g). The oil is dissolved in ether and hydrochloric acid in ether is added. The tan solid is recrystallized from methanol and ether to give a gray solid (0.21 g, m.p. 252°C).

Preparation A12(c): ***trans*-7-aminosulfonyl-5a,10b-dihydro-3N-n-propyl-6H-indeno[1,2-d]azepine,** see CHART A, STEP 12, compound A13.

The title compound is prepared from trans-7-aminosulfonyl-5a,10b-dihydro-6H-indeno[1,2-d]azepine in a manner similar to that for the preparation of trans-10-aminosulfonyl-5a,10b-dihydro-3N-n-propyl-6H-indeno[1,2-d]azepine, see preparation A11(c).

<u>PREPARATIONS AND EXAMPLES FROM CHART B</u>

STEP I

Preparation B1(a): **4-Chloro-7-methoxy-1-(N-pyrrolidinyl)indene,** see CHART B, STEP 1, compound B2.

A solution of the 4-chloro-7-methoxy-1-indanone (35.00 g, 0.178 mol) and pyrrolidine (76.0 g, 1.07 mol) in dry tetrahydrofuran (800 ml) is cooled to 7°C, and a solution of titanium tetrachloride (18.6 g, 0.098 mol) in pentane (50 ml) is added over a period of 7 minutes. The mixture is stirred at 10°C for 30 minutes at room temperature for 3.5 hours. The mixture is filtered, and the filtrate is evaporated under vacuum to leave the title compound as a green oil (44.4 g).

STEP 2

Preparation B2(a): **2-(Carboethoxymethyl)-4-chloro-7-methoxy-1-indanone,** see CHART B, STEP 2, compound B3.

A solution of the 4-chloro-7-methoxy-1-(N-pyrrolidinyl)indene (44.4 g, 0.178 mol), ethyl bromoacetate (32.7 g, 0.196 mol), and diisopropylethylamine (34.5 g, 0.267 mol) in acetonitrile (150 ml) is stirred at reflux in an oil bath maintained at 95°C for 4 hours and at room temperature for 15 hours. The mixture is titrated with water, and 10% hydrochloric acid is added to adjust the pH to 4-5. The mixture is heated on the steam bath for 15 minutes, cooled, and extracted with diethylether and with 1:1 diethylether/tetrahydrofuran. The combined extracts are washed with 10% hydrochloric acid (3 times), saturated sodium bicarbonate, and brine. The solution is dried ($MgSO_4$), and the solvent is removed under vacuum to leave an amber oil (41.3 g). Crystallization from ethyl acetate/hexane gives title compound as yellow crystals (28.9 g). The filtrate is purified by flash chromatography (230-400 mesh silica gel, 4:1 hexane/ethyl acetate) to give title compound as a solid (4.70 g, total yield 68%).

STEP 3

Preparation B3(a): **1,2-Bis(carboethoxymethyl)-4-chloro-2-hydroxy-7-methoxyindane,** see CHART B, STEP 3, compound B4.

A few crystals of iodine were added to a mixture of 2-(carboethoxymethyl)-4-chloro-7-methoxy-1-indanone (8.48 g, 30.0 mmol), ethyl bromoacetate (10.0 g, 60.0 mmol), and zinc dust (5.89 g, 90.1 mmol) in diethylether (50 ml) and benzene (100 ml) at room temperature, and the mixture is heated to reflux. After 30 minutes, no significant reaction occurred. A few more crystals of iodine are added, and the reflux is continued. The reaction is started after 30 minutes, and the mixture is refluxed for an additional 1.5 hours. The mixture is cooled, diluted with water, and washed with 10% hydrochloric acid, saturated sodium bicarbonate, and brine. The solution is dried ($MgSO_4$), and the solvent is removed under vacuum to leave the compound, B4(a), as an oil (10.89 g).

STEP 4

Preparation B4(a): **1,2-Bis(carboethoxymethyl)-4-chloro-7-methoxyindane,** see CHART B, STEP 4, compound B5.

Trifluoroacetic acid (80 ml) is added to a mixture of 1,2-bis(carboethoxymethyl)-4-chloro-2-hydroxy-7-methoxyindane (9.75 g, 0.026 mol) and triethylsilane (6.1 g, 0.052 mol) at room temperature with stirring. The mixture is stirred at room temperature for 3 hours, and the solvent is removed under vacuum. The material is dissolved in diethylether and washed with saturated sodium bicarbonate (twice) and brine. The solvent is removed under vacuum to leave an oil (8.9 g). Purification by flash chromatography (230-400 mesh silica gel, 10-20% ethyl acetate in hexane) gives the title compound as a mixture of cis-trans isomers (1.57 g).

STEP 5

Preparation B5(a): **1,2-Bis(2-hydroxyethyl)-4-chloro-7-methoxyindane,** see CHART B, STEP 5, compound B6.

A solution of 1,2-bis(carboethoxymethyl)-4-chloro-7-methoxyindane (1.72 g, 4. 85 mmol) in diethylether (50 ml) is added to a suspension of lithium aluminum hydride (1.5 g, 39.5 mmol) at room temperature. The mixture is stirred for 45 minutes, cooled in ice, and water (1.5 ml), 15% sodium hydroxide (1.5 ml), and water (4.5 ml) were added in succession. The mixture is stirred at room temperature for 15 minutes, and the precipitate is filtered and washed with tetrahydrofuran. The filtrate is dried ($MgSO_4$), and the solvent is removed under vacuum to leave the title compound (1.34 g) as a mixture of cis-trans isomers.

STEP 6

Preparation B6(a): **1,2-Bis(*p*-toluenesulfonyloxyeth-2-yl)-4-chloro-7-methoxyindane,** see CHART B, STEP 6, compound B7.

A solution of the 1,2-bis(2-hydroxyethyl)-4-chloro-7-methoxyindane (1.57 g, 5. 80 mmol) is cooled in ice, and p-toluenesulfonyl chloride (3.32 g, 17.4 mmol) is added. The mixture is stirred at 0°C for 1 hour and allowed to stand at 0°C for 20 hours. Water (12 ml) is added, and the mixture is stirred at room temperature for 45 minutes. The mixture is diluted with diethylether, washed with 10% hydrochloric acid (3 times), saturated sodium bicarbonate, and brine. The solution is dried ($MgSO_4$), and the solvent is removed under vacuum to leave the title compound as a yellow oil (2.9 g).

STEP 7

Preparation B7(a), Examples 11-12: preparation of the diastereomers, ***cis and trans*****-7-chloro-10-methoxy-5a,10b-dihydro-3N-*n*-propyl-6H-indeno[1,2-d]azepine,** seeCHARTB,STEP 7, compound B8.

A mixture of 1,2-bis(*p*-toluenesulfonyloxyeth-2-yl)-4-chloro-7-methoxyindane (2.9 g, 5.0 mmol), n-propylamine (0.33 g, 5.6 mmol), and potassium carbonate (2.07 g, 15.0 mmol) in acetonitrile is stirred at reflux in an oil bath maintained at 90°C for 3 hours. n-Propylamine (0.72 g, 12.2 mmol) is added, and the reflux continued for 19 hours. The mixture is diluted with diethylether and washed with water, 10% sodium carbonate, and brine. The solution is dried ($MgSO_4$), and the solvent is removed under vacuum to leave an oil (1.45 g). Purification by gravity chromatography (70-230 mesh silica gel, 0.7% isopropylamine and 10% ethyl acetate in hexane) gives two compounds. The faster moving compound (0.70 g) is dissolved in diethylether and acidified with excess ethereal hydrochloric acid. The precipitate is centrifuged, washed with diethylether, and crystallized from methanol/diethylether to give the trans-compound as a colorless solid (0.69 g, m.p. 195-196°C). The slower moving compound (0.55 g) is converted to the hydrochloride salt in a similar manner giving the cis-compound as an off-white solid (0.47 g, m.p. 245-246°C).

CHART A page 1

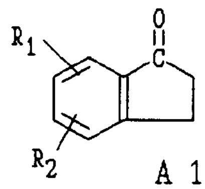

A 1

Step 1

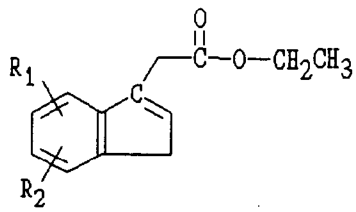

A 2

Step 2

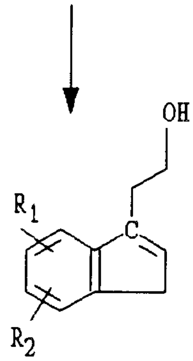

A 3

Step 3

CHART A page 2

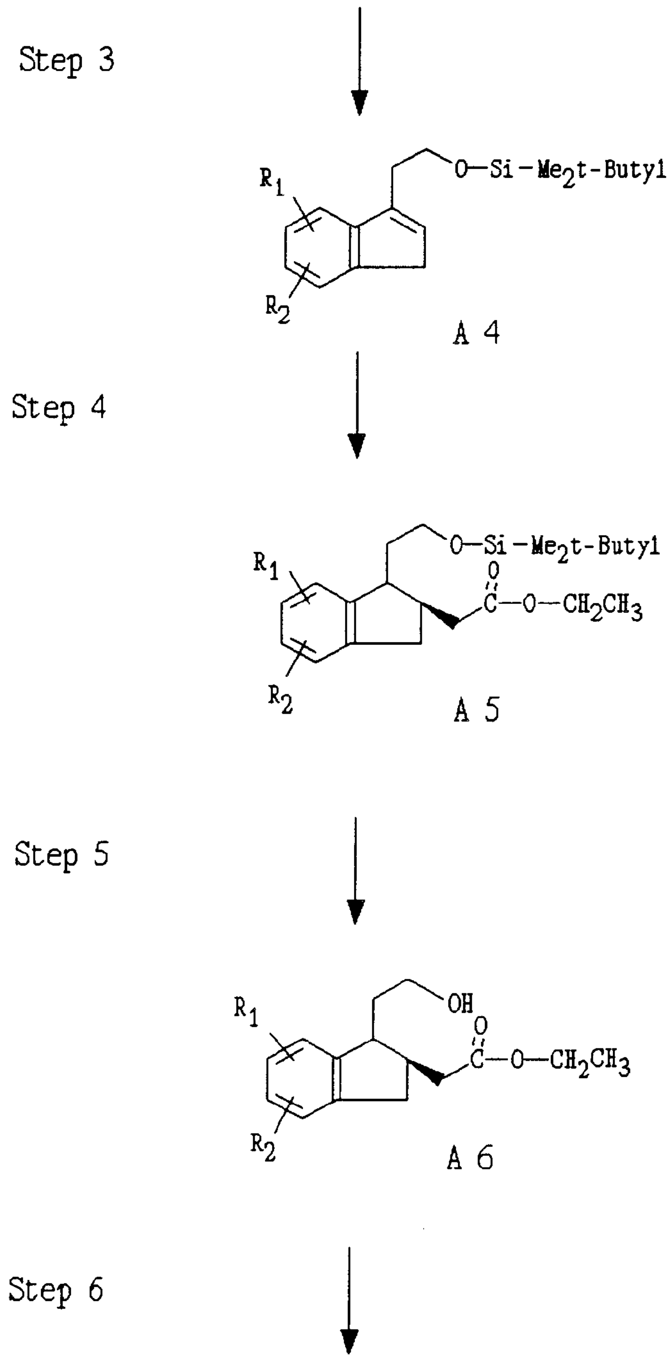


A 4

A 5

A 6

CHART A page 3

Step 6

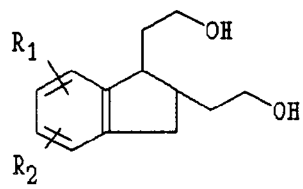

A 7

Step 7

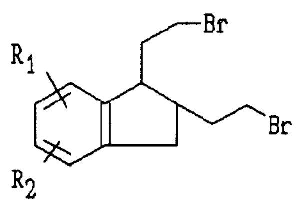

A 8

Step 8

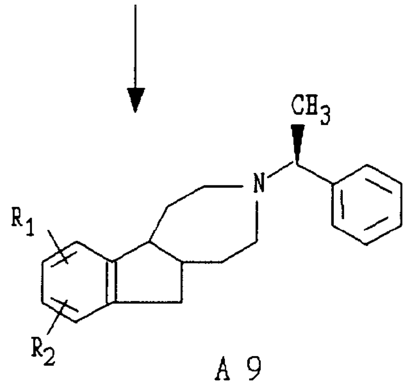

A 9

Step 9

CHART A page 4

Step 9

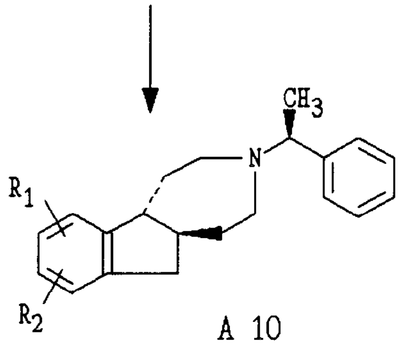

A 10

Step 10

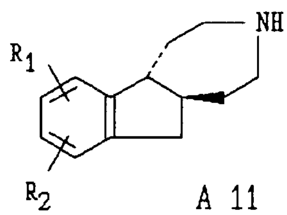

A 11

Step 11

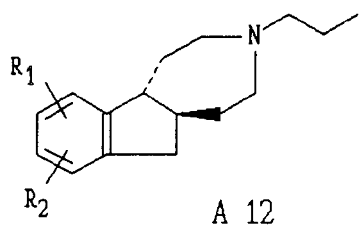

A 12

Step 12

CHART A page 5

Step 12

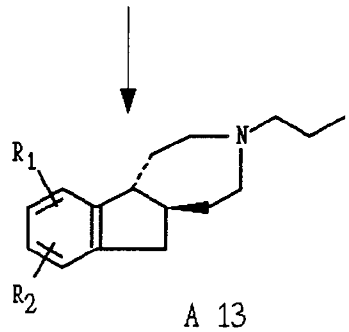

A 13

CHART B page 1

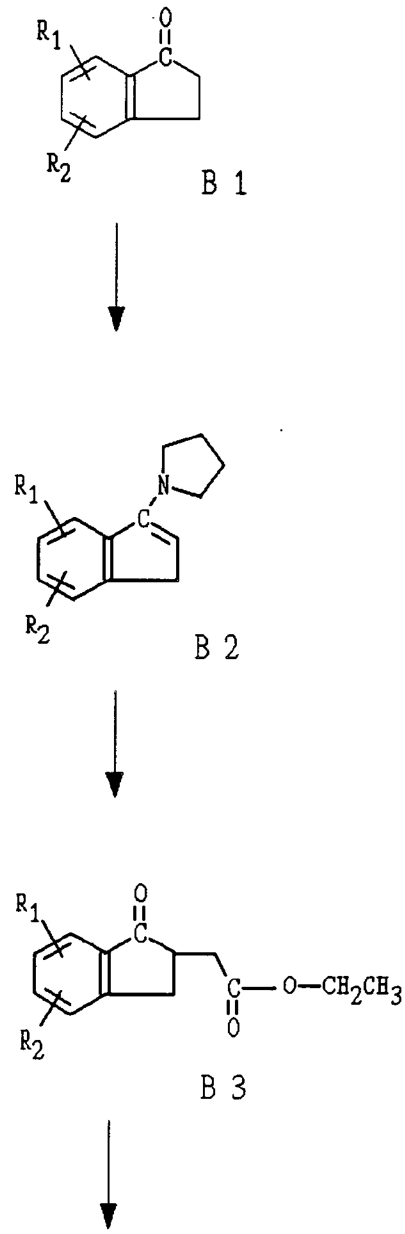

B 1

Step 1

B 2

Step 2

B 3

Step 3

CHART B page 2

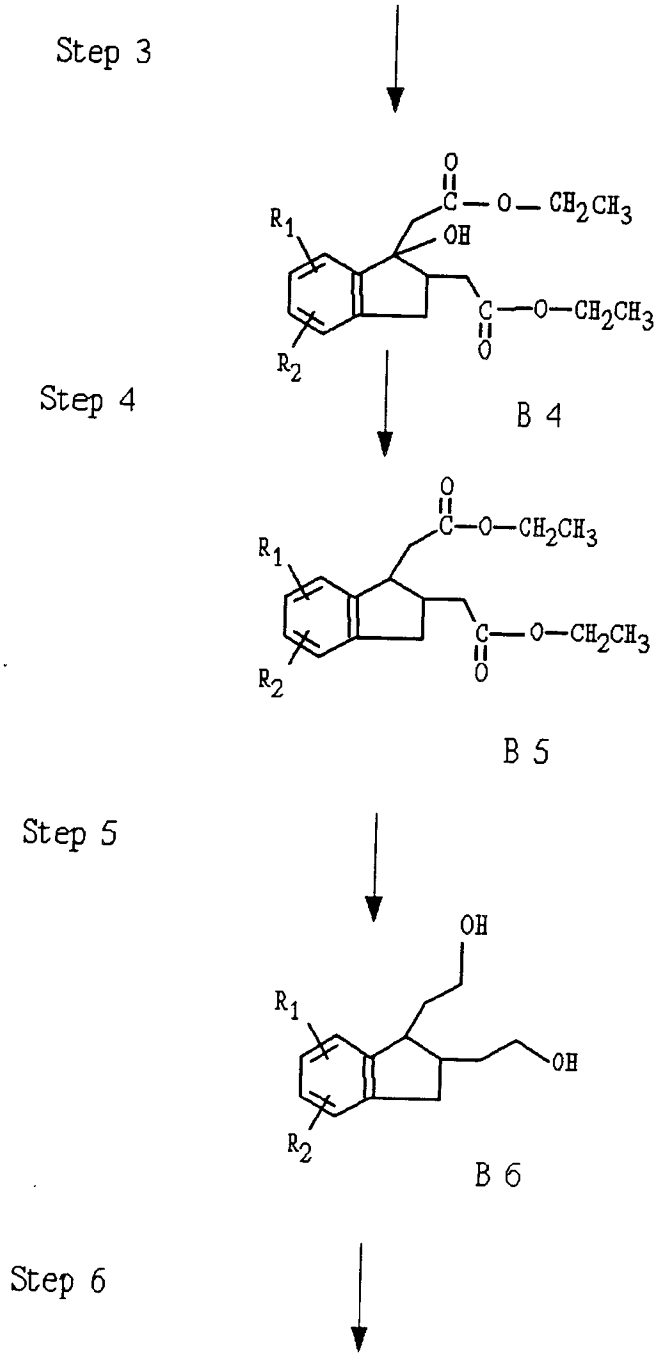


B 4

B 5

B 6

CHART B page 3

Step 6

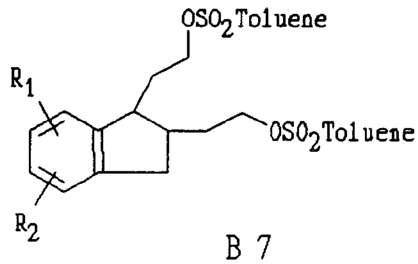

B 7

Step 7

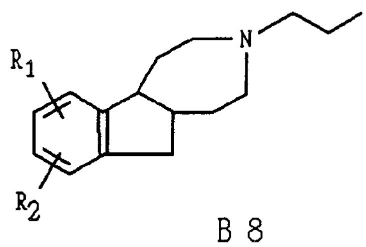

B 8

PART II

Reactions of CHART C

Step 1, carboxylic acid, C1, is reduced with borane/dimethylsulfide to give the benzylic alcohol, C2. Step 2, the benzylic alcohol, C2, is converted into the benzylic bromide, C3, with NBS and triphenylphosphine. Step 3, the benzylic bromide, C3 is alkylated with the enolate of ethyl acetate to give C4. Step 4, C4 is reduced to the alcohol, C5, with LAH. Step 5, the alcohol, C5, is oxidized under Swern conditions to afford the aldehyde, C6.

Step 6, the aldehyde, C6, is treated with vinylmagnesium bromide to afford the allylic alcohol, C7. Step 7, the allylic alcohol, C7, is treated with triethylorthoacetate to cause a Claisen rearrangement to give the ester, C8. Step 8, the ester, C8, is saponified with sodium hydroxide in aqueous methanol to afford the acid, C9. Step 9, the acid, C9, underwent a Curtus rearrangement with the aid of diphenylphosphorylazide to afford the carbamate, C10.

Step 10, the carbamate, C10, is cyclized to the 1,2,3,4,4a,5,6,10b *trans*octahydrobenzo[f]isoquinoline, C11, using paraformaldehyde and borontrifluoride-etherate. Step 11, the carbamate, C11, is saponified by refluxing potassium hydroxide in aqueous ethanol to afford the amine C12. Step 12, the amine, C12, is resolved into pure enantiomers by crystallization with optically active di-*p*-toluoyltartaric acid in methanol. From this point, step 12, each enantiomer is independently converted through the subsequent steps to afford optically pure products.

Step 13, the amine, now the pure enantiomer, C13, is converted into the propionamide, C14, with propionylchloride. Step 14, the propionamide, C14, is reduced with LAH to give C15. Alternatively, from Step 13, the

amine, the pure enantiomer, C13, can be converted directly into C15 by alkylation with bromopropane. Step 15, C15 is converted into the carboxamide, C16, by subsequent treatment with *t*-butyllithium and trimethylsilylisocyanate. The hydro-debrominated analog, C17, was isolated as a side product. The hydro-debrominated analog, C17, can be synthesized in high yield by treatment with *t*-butyllithium followed by a water quench.

Step 16, the carboxamide, C16, is dehydrated to the nitrile, C20, with the aid of Burgess' reagent. Step 17, the carboxamide, C16, is hydrogenated to remove the chlorine using palladium/carbon, affording C18. Step 18, the compound, C18, is dehydrated using Burgess' reagent to afford the nitrile, C19.

Structures are shown in CHART C.

The compounds of this invention have high oral potency and a long duration of action. This exceptionally good bioavailability combined with a long period of activity are beneficial to effective clinical treatment. The preferred compounds of PART II are not only active but have increased bioavailability as shown by metabolism studies. See PART II - TABLES I-II, below. The preferred compounds from the list below and PART II - TABLES I and II are compounds numbered 1, 2, 7, and 11.

The compounds of this invention are useful both as intermediates to produce other compounds and they are useful to treat central nervous system disorders. The utility of the compounds of this invention to treat central nervous system disorders is shown in behavioral, physiological and biochemical tests. These tests are described below.

CNS Receptor Binding Assay (PART II - TABLE I): The Central Nervous System Binding Assay measures the percent inhibition from experiments employing test compounds at 1 microMolar concentration competing with various radioligands for binding to whole brain membranes, membranes prepared from specific brain structures, or membranes prepared from cell lines expressing cloned receptors. When percent inhibition is equal to 100 the test compound binds as well as the standard compound. The standard compound for the dopamine D1 receptor is SCH 23390.

Metabolism (PART II - TABLE II):

Hepatocyte. Intrinsic clearance of 3 concentrations of compound (2, 5 and 15 ug/ml) following a 60 minute incubation at 37°C in the presence of a suspension of freshly prepared rat hepatocytes (5.0 million cells/ml). Aliquots of each incubate were withdrawn during the incubation and analyzed for parent compound using HPLC methodology. Intrinsic clearance is expressed as ml/min/5 million cells. Metabolic stability relative to the control, (±)1-formyl-6,7,8,9-tetrahydro-N-di-n-propyl-8-amino-3H-benz[e]indole, is determined.

Microsome. Micromes prepared from Sprague Dawley rat livers were incubated at 37 C with 5 or 12.5 micromolar substrate. The metabolic half-lives of the test compounds were determined from the HPLC from plots of LN(peak area) vs time. These were ratioed with the correspondinghalf-lifeof5-(dipropylamino)-5,6-dihydro-4H-imidazo(4,5,1-ij)-quinoline-2(1H)-one, (-), monohydrobromide hydrate to give relative metabolic half lives.

The compounds below have been subjected to one or more of the biological tests described above. PART II - TABLES I - II use the numbers assigned below.

| Compound Number | Name |
|---|---|
| 1 | **(-)-10-Bromo-7-chloro-1,2,3,4a,5,6,10b-*trans*-octahydro-3N-propylbenzo[f]isoquinoline,** See CHART C, STEP 14, compound C15, EXAMPLE 1. |
| 2 | **(+)-10-Bromo-7-chloro-1,2,3,4,4a,5,6,10b-*trans*-octahydro-3-N-propylbenzo[f]isoquinoline,** See CHART C, STEP 14, compound C15, EXAMPLE 2. |
| 3 | **(-)-7-Chloro-10-carboxamido-1,2,3,4,4a,5,6,10b-*trans*-octahydro-3N-propylbenzo[f]isoquinoline,** See CHART C, STEP 15, compound C16, EXAMPLE 3. |
| 4 | **(+)-7-Chloro-10-carboxamido-1,2,3,4,4a,5,6,10b-*trans*-octahydro-3N-propylbenzo[f]isoquinoline,** See CHART C, STEP 15, compound C16, EXAMPLE 4. |
| 5 | **(-)-7-Chloro-1,2,3,4,4a,5,6,10b-*trans*-octahydro-3N-propylbenzo[f]isoquinoline,** compound C17, from preparation C15, EXAMPLE 5. |
| 6 | **(+)-7-Chloro-1,2,3,4,4a,5,6,10b-*trans*-octahydro-3N-propylbenzo[f]isoquinoline,** compound C17, from preparation C15, EXAMPLE 6. |
| 7 | **(-)-7-Chloro-10-cyano-1,2,3,4,4a,5,6,10b-*trans*-octahydro-3N-propylbenzo[f]isoquinoline.** see CHART C, STEP 16, compound C20, EXAMPLE 7. |
| 8 | **(+)-7-Chloro-10-cyano-1,2,3,4,4a,5,6,10b-*trans*-octahydro-3N-propylbenzo[f]isoquinoline.** see CHART C, STEP 16, compound C20, EXAMPLE 8. |
| 9 | **(-)-10-Carboxamido-1,2,3,4,,4a,5,6,10b-ocathydro-*trans*-3N-propylbenzo[f]isoquinoline**, see CHART C, STEP C17, compound C18, EXAMPLE 9. |
| 10 | **(+)-10-Carboxamido-1,2,3,4,4a,5,6,10b-octahydro-*trans*-3N-propylbenzo[f]isoquinoline,** see CHART C, STEP 17, compound C18, EXAMPLE 10. |
| 11 | **(-)-10-Cyano-1,2,3,4,4a,5,6,10b-octahydro-*trans*-3N-propylbenzo[f]isoquinoline,** see CHART C, STEP 18, compound C19, EXAMPLE 11. |
| 12 | **(+)-10-Cyano-1,2,3,4,4a,5,6,10b-octahydro-*trans*-3N-propylbenzo[f]isoquinoline,** see CHART C, STEP 18, compound C19, EXAMPLE 12. |

PART II - TABLE I

BIOLOGICAL DATA - CNS Receptor Binding

| Compound No. | Dopamine - D1 % inhibition | Dopamine - D2 % inhibition |
|---|---|---|
| 1 | 92.09 | 97.06 |
| 2 | 16.00 | 81.64 |
| 3 | 14.54 | 43.42 |
| 4 | 3.18 | 27.37 |
| 5 | 25.08 | 89.87 |
| 6 | 40.95 | 95.92 |
| 7 | 83.17 | 100.00 |
| 8 | 9.40 | 24.67 |
| 9 | 24.77 | 2.90 |
| 10 | 48.35 | 4.87 |
| 11 | 36.66 | 100.00 |
| 12 | 15.14 | 90.18 |

PART II - TABLE II

BIOLOGICAL DATA - Metabolism (hepatocyte and microsome)

| Compound No. | Hepatocyte (control is 1.0) | Microsome (control is 1.0) |
|---|---|---|
| 1 | 0.120 | -- |
| 2 | 0.140 | -- |
| 3 | -- | -- |
| 4 | -- | -- |
| 5 | 0.120 | 0.20 |
| 6 | 0.040 | -- |
| 7 | 0.230 | 0.91 |
| 8 | 0.200 | -- |
| 9 | 0.500 | 1.30 |
| 10 | 1.610 | -- |
| 11 | 0.130 | 0.48 |
| 12 | 0.070 | 0.28 |

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

PREPARATIONS AND EXAMPLES FROM CHART C

STEP 1

Preparation C1, **Bromo-2-chlorobenzyl alcohol**, see CHART C, STEP 1, compound C2.

5-Bromo-2-chlorobenzoic acid (107.9 g, 458.2 mmol) in THF (1000 mL) is cooled to 0°C. Borane-methyl sulfide complex (1.3 eq) is added. After 25 minutes at room temperature the solution is refluxed for 30 minutes. Water, 300 mL, is added at 0°C, then 2N hydrochloric acid is added. After stirring 45 minutes at room temperature, additional acid is added and the mixture extracted with ether/methylene chloride. The organic layer is washed successively with more aqueous acid, water, saturated aqueous sodium bicarbonate and brine. The organic layer is then dried over sodium sulfate, filtered, and stripped of solvent to yield a white solid, 100.9 g (99%), mp 90.0-92.0°C.

STEP 2

Preparation C2, **5-Bromo-2-chlorobenzyl bromide,** see CHART C, STEP 2, compound C3.

5-Bromo-2-chlorobenzyl alcohol (106.6 g, 481.4 mmol) in methylene chloride (1000 mL) is cooled to 0°C. N-Bromo-succinimide (1.2 eq) is added over 60 minutes and the ice bath is removed. After 2 hours, methanol (8 mL) is added. The solution is poured into 40% ether in hexane (2100 mL); silica gel (800 mL, 230-400 mesh) is added. The mixture is filtered through another 200 mL of silica gel and the silica gel plug is rinsed with 40% ether in hexane (1050 mL). Solvents are removed and the residue is flash chromatographed on a silica gel column (24 x 7 cm), followed by elution with methylene chloride/ethyl acetate/hexane (2:10:88) to yield a red solid, 108.9 g (79%), mp 72.5-74.5°C.

STEP 3

Preparation C3, **ethyl 3-(5-bromo-2-chlorophenyl)propionate**, see CHART C, STEP 3, compound C4.

5-Bromo-2-chlorobenzyl bromide (108.9 g, 382.9 mmol) in THF (475 mL) is added to ethyl acetate (60.0 mL, 612.7 mmol) in lithium diisopropylamide (1.5 eq in THF, 650 mL) at - 78°C. After stirring for 2 hours, glacial acetic acid (1 equivalent) is added. The mixture is partitioned between ether and 2N hydrochloric acid. The organic layer is washed with water, saturated aqueous sodium bicarbonate, and brine. It is dried over sodium sulfate, filtered, and the solvents removed under vacuum. The residual oil is flash chromatographed on a 20x7 cm silica gel column. The silica gel column is eluted with 7.5% ethyl acetate in hexane to yield 87.2 g (78 %) orange oil.

STEP 4

Preparation C4, **3-(5-Bromo-2-chlorophenyl)propanol,** see CHART C, STEP 4, compound C5.

Ethyl 3-(5-bromo-2-chlorophenyl)propionate (87.2 g, 299.1 mmol) in ether (300 mL) is added to lithium aluminum hydride (12.0 g, 299.1 mmol) in ether (300 mL) at 0°C. After 30 minutes, water (10 mL), sodium hydroxide (10 mL, 15% aqueous), and water (30 mL) are added successively and stirred at room temperature for 15 minutes. The slurry is filtered and the filtrate is dried over sodium sulfate to yield a pale orange oil (68.9 g, 92%).

STEP 5

Preparation C5, **3-(5-Bromo-2-chlorophenyl)propionaldehyde,** see CHART C, STEP 5, compound C6.

Dimethyl sulfoxide (43.1 mL, 0.61 M) in methylene chloride (300 mL) is cooled to -78°C. Oxalyl chloride (26.5 mL, 0.30 M) is added. After 30 minutes, 3-(5-bromo-2-chlorophenyl)propanol (68.9 g, 0.28 M) in methylene chloride (250 mL) is added. After 20 minutes, triethylamine (192 mL, 1.38 M) is added. The slurry is warmed to room temperature. Water is added and the mixture is extracted with ether. The organic layer is successively washed with 2N hydrochloric acid, water, saturated aqueous sodium bicarbonate, and brine. It is dried over sodium sulfate and yields 68.3 g (100%) pale oil.

STEP 6

Preparation C6, **5-(5-Bromo-2-chlorophenyl)penten-3-ol**, see CHART C, STEP 6, compound C7.

Vinylmagnesium bromide (339 mmol) in THF (900 mL) is cooled to 0°C. 3-(5-Bromo-2-chlorophenyl) propionaldehyde (68.3 g, 276 mmol) in THF (500 mL) is added and the solution stirred at room temperature for 2.5 hours. Saturated aqueous ammonium chloride is added, then 2N hydrochloric acid is added. The mixture is extracted with ether. The ether layer is washed with water then with brine. The washed ether layer is dried over sodium sulfate and yielded 71.6 g (94%) yellow oil.

STEP 7

Preparation C7(a), **ethyl 7-(5-bromo-2-chlorophenyl)-*trans*-hept-4-enoate,** see CHART C, STEP 7, compound C8.

5-(5-Bromo-2-chlorophenyl)penten-3-ol (71.6 g, 260 mmol), triethylorthoacetate (164 mL, 885 mmol), and propionic acid (1.1 mL, 0.99 mmol) are combined and heated at 150°C for 2 hours with removal of ethanol. The solution is cooled to 0°C and 2N hydrochloric acid is added. The mixture is extracted with ether. The ether layer is washed with water then with brine. The either layer is then dried over sodium sulfate and yields 84.12 g (94%) of yellow oil.

STEP 8

Preparation C8, **7-(5-Bromo-2-chlorophenyl)-*trans*-hept-4-enoic** acid, see CHART C, STEP 8 compound C9.

Ethyl 7-(5-bromo-2-chlorophenyl)-*trans*-hept-4-enoate (84.1 g, 243 mmol), potassium hydroxide (47 g, 730 mmol), methanol (400 mL), and water (50 mL) are refluxed for 2.5 hours. The solvents are removed under vacuum and the residue is partitioned between ether and water. The aqueous layer is acidified with hydrochloric acid and extracted with ether. The ether layer is washed with water and brine and is dried over sodium sulfate. It yields 57.8 g (75%) yellow solid. A portion is recrystallized from ethyl acetate, mp 53.5-55.0°C.

STEP 9

Preparation C9, **6-(5-Bromo-2-chlorophenyl)-*trans*-hex-3-enyl ethyl carbamate**, see CHART C, STEP 9, compound C10.

7-(5-Bromo-2-chlorophenyl)-*trans*-hept-4-enoic acid (57.8 g, 182 mmol), 1,4-dioxane (600 mL), diphenylphosphoryl azide (39.2 mL, 182 mmol),and triethylamine (25.4 mL, 182 mmol) are stirred at room temperature for 1 hour, then refluxed 30 minutes. Nitrogen evolves. Ethanol (22.1 mL, 364 mmol) is added and refluxing continues for 2 hours. The solution is cooled to 0°C and sodium hydroxide (1.1 equivalent) and water are added. After 30 minutes, the mixture is partitioned between ether and water. The ether layer is washed successively with water, 2N hydrochloric acid, water, saturated aqueous sodium bicarbonate and brine. The solution is dried over sodium sulfate. The yellow oil is flash chromatographed on a 16 x 7 cm silica gel column and eluted with 5% followed by 10% ethyl acetate in hexane to yield 37.2 g (57%) yellow oil.

STEP 10

Preparation C10, **10-Bromo-3N-carboethoxy-7-chloro-1,2,3,4,4a,5,6,10b-*trans*-octahydrobenzo[f]isoquinoline,** see CHART C, STEP 10, compound C11.

6-(5-Bromo-2-chlorophenyl)-*trans*-hex-3-enyl ethyl carbamate (46.4 g, 129 mmol) is dissolved in methylene chloride (250 mL). Paraformaldehyde (4.3 g, 135 mmol) is added and then boron trifluoride etherate (17.4 mL, 142 mmol). The slurry is warmed to 30°C and after 5 minutes it is poured onto sodium carbonate and ice. The slurry is extracted with ether. The ether layer is washed with brine and then dried over sodium sulfate. This produces a thick orange oil which is flash chromatographed on a 19 x 7 cm silica gel column and eluted with 2% acetone in hexane to yield 41.6 g (83 %) of pale oil.

STEP 11

Preparation C11, **10-Bromo-7-chloro-1,2,3,4,4a,5,6,10b-*trans*-octahydrobenzo[f]isoquinoline,** See CHART C, STEP 11, compound C12.

10-Bromo-3N-carboethoxy-7-chloro- 1,2,3,4,4a,5,6,10b-*trans*-octahydrobenzo[f]isoquinoline (41.1 g, 110 mmol) is refluxed with potassium hydroxide (28.5 g, 441 mmol), methanol (200 mL), and water (20 mL) for 65 hours. The solvents are removed under vacuum and the residue is partitioned between water and ether/methylene chloride. The organic layer is washed with water twice and then with brine. The organic layer is dried over sodium sulfate to yield 30.9 g (93% crude) of pale oil.

STEP 12

Preparation C12, **enantiomeric separation of 10-bromo-7-chloro-1,2,3,4,4a,5,6,10b-*trans*-octahydrobenzo[f]isoquinoline,** see CHART C, STEP 12, compound C13.

A hot methanol solution (650 mL) of 10-bromo-7-chloro-1,2,3,4,4a,5,6,10b-*trans*-octahydrobenzo[f]isoquinoline (30.9 g, 103 mmol) is combined with di-*p*-toluoyl-D-tartaric acid (22.5 g, 56.5 mmol). Upon cooling, the resultant white solid is filtered. The filtrate is dried of solvent under vacuum, then partitioned between aqueous sodium hydroxide and ether. The ether layer is washed with water and brine, then dried over sodium sulfate to yield a pale oil. The above procedure is repeated five more times alternating between the D and the L forms of the acid. The combined D-salts are then converted to free base and converted back to the D-salts as described above. A second cycle yielded the free base as a white waxy solid, 9.0 g (29%). $[\alpha]^{25}_{589}$= -212.9° (c= 0.68, MeOH).

NOTATION COMMENT

Steps 13 - 19, below, show how the enantiomers, "(a)" and "(b)" are independently converted to give optically pure products. Enantiomers for aminosulfonyl derivatives are labeled with preparation numbers that include the letters "(c)" and "(d)."

STEP 13

Preparation C13(a), **(-)-10-Bromo-7-chloro-1,2,3,4,4a,5,6,10b-*trans*-octahydro-3N-propionylbenzo [f]isoquinoline**, See Chart C, STEP 13, compound C14.

(-)-10-Bromo-7-chloro-1,2,3,4,4a,5,6,10b-*trans*-octahydro benzo[f]isoquinoline (1.75 g, 5.82 mmol), triethylamine (0.97 mL, 6.98 mmol), and methylene chloride (12 mL) are combined and cooled to 0°C. Propionyl chloride (0.57 mL, 6.40 mmol) is added. After 1 hour water is added and the mixture is extracted with ether. The ether layer is washed with 2N hydrochloric acid, water, saturated aqueous sodium bicarbonate, and brine. The ether layer is dried over sodium sulfate to yield 2.17 g (100%) thick oil.

Preparation C13(b), **(+)-10-Bromo-7-chloro-1,2,3,4,4a,5,6,10b-*trans*-octahydro-3N-propionylbenzo [f]isoquinoline**, See Chart C, STEP 13, compound C14.

Use the same procedure as above, for C13(a), only start with (+)-10-Bromo-7-chloro-1,2,3,4,4a,5,6,10b-*trans*-octahydro benzo[f]isoquinoline.

STEP 14

Preparation C14(a), EXAMPLE 1, **(-)-10-Bromo-7-chloro-1,2,3,4,4a,5,6,10b-*trans*-octahydro-3N-propylbenzo[f]isoquinoline,** See CHART C, STEP 14, compound C15.

(-)-10-Bromo-7-chloro-1,2,3,4,4a,5,6,10b-*trans*-octahydro-3N-propionylbenzo[f]isoquinoline (2.17 g, 6.08 mmol) in ether (6 mL) is added to lithium aluminum hydride (0.23 g, 5.82 mmol) in ether (6 mL) at 0°C. The mixture is refluxed for 1 hour, then water (0.20 mL), sodium hydroxide (0.20 mL, 15% aqueous), and water (0.60 mL) are sequentially added. The mixture is filtered and the filtrate is dried over sodium sulfate to yield 1.88 g thick oil. The oil is flash chromatographed on a 2.2 x 15 cm silica gel column and eluted with 15 % ethyl

acetate in hexane to yield 1.77 g (89%) of an oil. $[\alpha]^{25}_{589}$= -176.55° (c= 1.70 $CHCl_3$). The oil is dissolved in ether and is acidified with hydrochloric acid in ether. The resulting white solid is recrystallized from methanol and ether to yield a white solid, m.p. 277°C (decomposition).

Preparation C14(b), EXAMPLE 2, **(+)-10-Bromo-7-chloro-1,2,3,4,4a,5,6,10b-*trans*-octahydro-3N-propylbenzo[f]isoquinoline,** See CHART C, STEP 14, compound C15.

Use the same procedure as above, for C14(a), only start with (+)-10-Bromo-7-chloro-1,2,3,4,4a,5,6,10b-*trans*-octahydro-3N-propionylbenzo[f]isoquinoline.

STEP 15

Preparation C15(a), EXAMPLE 3, **(-)-7-chloro-10-carboxamido-1,2,3,4,4a,5,6,10b-*trans*-octahydro-3N-propylbenzo[f]isoquinoline,** See CHART C, STEP 15, compound C16.

(-)-10-Bromo-7-chloro-1,2,3,4,4a,5,6,10b-*trans*-octahydro-3N-propylbenzo[f]isoquinoline (1.20 g, 3.50 mmol) in THF (10 mL) is cooled to -78°C and t-butyllithium (4.1 mL, 1.7 M in pentane) is added. Trimethylsilylisocyanate (1.1 mL, 7.00 mmol) is added. After 75 minutes at room temperature, water is added and the mixture extracted with ether. The ether layer is washed with saturated aqueous sodium bicarbonate and brine. The washed ether layer is dried over sodium sulfate. The crude product is flash chromatographed on a 2.2 x 15 cm silica gel column and eluted with 5:1 methylene chloride:methanol. Product obtained is 0.86 g (80 %) of a white solid, m.p. 220°C (decomposition). $[\alpha]^{25}_{589}$ = -255.82° (c=0.455 $CHCl_3$). This solid is dissolved in isopropanol and ether, then hydrochloric acid in ether is added. The white solid is recrystallized from isopropanol and ether, m.p. 165°C (decomposition).

Preparation C15(b), EXAMPLE 4, **(+)-7-Chloro-10-carboxamido-1,2,3,4,4a,5,6,10b-*trans*-octahydro-3N-propylbenzo[f]isoquinoline,** See CHART C, STEP 15, compound C16.

Use the same procedure as above, for C15(a), only start with (+)-10-bromo-7-chloro-1,2,3,4,4a,5,6,10b-*trans*-octahydro-3N-propylbenzo[f]isoquinoline.

EXAMPLE 5, isolation of **(-)-7-Chloro-1,2,3,4,4a,5,6,10b-*trans*-octahydro-3N-propylbenzo [f]isoquinoline**, compound C17, from preparation C15. This compound is isolated from (-)-7-chloro- 10-carboxamido-1,2,3,4,4a,5,6, 10b-*trans*-octahydro-3N-propylbenzo[f]isoquinoline chromatography using an initial elution of 20:1 methylene chloride:methanol to yield 0.30 g (1.14 mmol) pale paste. This is dissolved in methanol and ether and hydrochloric acid in ether is added. The white solid is recrystallized from methanol and ether to yield 0.14 g white solid, m.p. 250°C (decomposition), $[\alpha]^{25}_{589}$= -92.73° (c= 0.33 MeOH).

EXAMPLE 6, isolation of **(+)-7-Chloro-1,2,3,4,4a,5,6,10b-*trans*-octahydro-3N-propylbenzo [f]isoquinoline,** compound C17, from preparation C15. This compound is isolated from (-)-7-chloro-10-carboxamido-1,2,3,4,4a,5,6, 10b-*trans*-octahydro-3N-propylbenzo[f]isoquinoline using the same procedure as above.

Preparation C15(c) and C15(d) **(+) and (-)-7-chloro-10-aminosulfonyl-1,2,3,4,4a,5,6,10b-*trans* -octahydro-3N-propylbenzo[f]isoquinoline,** See CHART C, STEP 15, compound C16. The title sulfonamides are prepared from the enantiomers of 10-Bromo-7-chloro-1,2,3,4,4a,5,6, 10b-*trans* -octahydro-3N-propylbenzo[f]isoquinoline, compound C15, via metal-halogen exchange with t-butyllithium, followed by conversion of the aryllithium to the sulfonamide according to the reference: S.L. Graham, et al., J. Med. Chem., 32:2548 (1989).

STEP 16

Preparation C16(a), EXAMPLE 7, **(-)-7-Chloro-10-cyano-1,2,3,4,4a,5,6,10b-*trans*-octahydro-3N-propylbenzo[f]isoquinoline.** see CHART C, STEP 16, compound C20.

(-)7-chloro-1-carboxamido-1,2,3,4,4a,5,6,10b-*trans*-octahydro-3N-propylbenzo[f]isoquinoline (0.45 g, 1.47 mmol) is dissolved in THF (7.5 mL) and methylene chloride (9.0 mL) and Burgess' reagent (0.87 g, 3.67 mmol) are added. After 3 hours at room temperature it is poured onto 10% aqueous sodium carbonate at 0°C and extracted with ether. The ether layer is washed with brine and dried over sodium sulfate. The amber paste is flash chromatographed on a 1.2 x 16 cm silica gel column, eluted with 10:1 methylene chloride/methanol to yield 0.41 g (97%) opaque tan oil, $[\alpha]^{25}_{589}$= -209.6°, (c = 1.09 MeOH). This is dissolved in methanol and ether and hydrochloric acid in ether is added. The white solid is recrystallized from methanol and ether, m.p. 275°C

(decomposition).

Preparation C16(b), EXAMPLE 8, **(+)-7-Chloro-10-cyano-1,2,3,4,4a,5,6,10b-*trans*-octahydro-3N-propylbenzo[f]isoquinoline**. see CHART C, STEP 16, compound C20.

Use the same procedure as above, for C16(a), only start with (+)-7-chloro-10-carboxamido -1,2,3,4,4a,5,6, 10b-*trans*-octahydro-3N-propylbenzo[f]isoquinoline.

STEP 17

Preparation C17(a), EXAMPLE 9, **(-)-10-Carboxamido-1,2,3,4,4a,5,6,10b-octahydro-*trans*-3N-propylbenzo[f]isoquinoline**, see CHART C, STEP C17, compound C18.

(-)-7-Chloro-10-caboxamido-1,2,3,4,4a,5,6,10b-octahydro-*trans*-3N-propylbenzo[f]isoquinoline (1.5g) is placed in a hydrogenation flask with palladium hydroxide/carbon (0.45g) and ethanol (120 ml). This is hydrogenated under 50 p.s.i. for 12 hours then filtered through diatomaceous earth. Solvent evaporation is followed by dissolving the residue in methylene chloride and 10% aqueous sodium carbonate. The organic layer is separated and washed with water and then brine, and dried over sodium sulfate. Solvent removal affords a white solid. $[\alpha]^{25}_{589}$ = -307.80° (c = 0.68 methanol). The amine is converted into the crystalline maleate salt with maleic acid in methanol/ether (needles) $[\alpha]^{25}_{589}$ = -214.95° (c = 1.07 methanol), m.p. 240°C (decomposition).

PreparationC17(b), EXAMPLE 10, **(+)-10-Carboxamido-1,2,3,4,4a,5,6,10b-octahydro-*trans*-3N-propylbenzo[f]isoquinoline,** see CHART C, STEP 17, compound C18. Use the same procedure as above, for C17(a), only start with (+)-7-chloro-10-carboxamido-1,2,3,4,4a,5,6,10b-octahydro-*trans*-3N-propylbenzo[f]isoquinoline.

Preparation C 17(c) and C 17(d), **(+) and (-) -(10)-aminosulfonyl-1,2,3,4,4a,5,6,10b-trans-octahydro-3N-propylbenzo[f]isoquinoline,** see CHART C, STEP 17, compound C18.

STEP 18

PreparationC18(a),EXAMPLE 11,**(-)-10-Cyano-1,2,3,4,4a,5,6,10b-octahydro-*trans*-3N-propylbenzo [f]isoquinoline,** see CHART C, STEP 18, compound C19. (-)-10-Carboxamido-1,2,3,4,4a,5,6,10b-octahydro-*trans*-3N-propylbenzo[f]isoquinoline(300mg, 1.1 mmol) is dissolved in methylene chloride. Burgess' reagent (3 eq. 785 mg) is added and the solution is stirred for two hours. The solution is poured into 10 % aqueous sodium carbonate and extracted. The organic layer is washed with water and then brine. Drying over sodium sulfate and solvent removal affords an oil $[\alpha]^{25}_{589}$ = -202.64° (c = 1.89 chloroform). This is converted to the crystalline hydrochloride salt with etheral hydrogen chloride and methanol, m.p. 325°C (decomposition).

Preparation C18(b), EXAMPLE 12, **(+)-10-cyano-1,2,3,4,4a,5,6,10b-octahydro-*trans*-3N -propylbenzo[f]isoquinoline,** see CHART C, STEP 18, compound C19. Use the same procedure as above, for C18(a), only start with (+)-10-carboxamido-1,2,3,4,4a,5,6, 10b-octahydro-*trans*-3N-propylbenzo[f]isoquinoline.

The following alternative procedures are provided for the synthesis of some compounds. The following synthesis steps begin in place of step 13 and are numbered 131, 132 etc., see page 5 of CHART C.

STEP 131

Preparation C131, Example 13, **(-)-10-Bromo-7-chloro-1,2,3,4,4a,5,6,10b-*trans*-octahydro-3N-benzylbenzo[f]isoquinoline,** see CHART C, STEP 131, compound C 132. Begin with starting materials for preparation C13 from STEP 13 above. Benzyl chloride (5.4 mL, 45.4 mmol) is added to triethylamine (6.6 mL, 47.6 mmol), (-)-10-bromo-7-chloro-1,2,3,4,4a,5,6, 10b-*trans*-octahydrobenzo[f]isoquinoline (13.0 g, 43.24 mmol), and dimethylformamide (90 mL) at 0°C. After 30 minutes, the ice bath is removed and the slurry stirred at room temperature for two days. Water and ether are added. The organic layer is washed with water and brine and is dried with sodium sulfate to yield an amber oil. $[\alpha]^{25}_{589}$=-139.32° (c= 1.04, MeOH).

Notation comment. In the compounds of this series the preparations followed by a lower case (a) are where R = H, lower case (b) indicates R = $CH_3$.

STEP 132

PreparationC132(a), Example 14, **(-)-7-Chloro-10-sulfonamido-1,2,3,4,4a,5,6,10b-*trans*-octahydro-**

**3N-benzylbenzo[f]isoquinoline,** see CHART C, STEP 132, Compound C 133, R = H. *tert*-Butyllithium (8.0 mL, 1.7 M in pentane) is added to (-)-10-bromo-7-chloro-1,2,3,4,4a,5,6, 10b-*trans*-octahydro-3N-benzylbenzo[f]isoquinoline (2.58 g, 6.60 mmol) in THF (15 mL) at -78°C. After 10 minutes, sulfuryl chloride (0.8 mL, 9.90 mmol) is added in a quick dose and the cold bath removed. After 1 hour, the solvents are removed under vacuum. THF (15 mL) is added and the mixture cooled on an ice bath and ammonia saturated THF (15 mL) added in a quick dose to the slurry, and the ice bath removed. Ammonium hdyroxide (10 mL) is then added. After 2 hours, water and ether are added. The organic layer is washed with water and brine and dried over sodium sulfate to yield an amber foam, m.p. 97- 102°C. $[\alpha]^{25}_{589}$= -158.2° (c = 0.56, MeOH).

Preparation C 132(b), Example 15, **(-)-7-Chloro-10-methylsulfonamido-1,2,3,4,4a,5,6,10b-*trans*-octahydro-3N-benzylbenzo[f]isoquinoline**, see CHART C, STEP 132, Compound C133, R = $CH_3$. *tert*-Butyllithium (9.3 mL, 1.7 M in pentane) is added to (-)-10-bromo -7-chloro-1,2,3,4,4a,5,6, 10b-*trans*-octahydro-3N-benzylbenzo[f]isoquinoline (3.0 g, 7.68 mmol) in THF (7.5 mL) at -78°C. After 10 minutes, sulfuryl chloride (2.6 mL, 15.8 mmol) is added in a quick dose and the cold bath removed. THF (7.5 mL) is added and after 1 hour, the solvents are removed under vacuum. THF (15 mL) is added and methylamine saturated THF is added. After 50 minutes, water and ethyl acetate are added. The organic layer is washed with water and brine and dried over sodium sulfate to yield a yellow foam. The foam is flash chromatographed on a 19 x 2 cm silica gel column and eluted with 10% methylene chloride and 25% ethyl acetate in hexane to yield a yellow foam, m.p. 99°C. $[\alpha]^{25}_{589}$= -148.5° (c= 0.60, MeOH).

STEP 133

Preparation 133(a), Example 16, **(-)-10-Sulfonamido-1,2,3,4,4a,5,6,10b-*trans*-octahydrobenzo[f]isoquinoline hydrochloride,** see CHART C, STEP 133, Compound C 134, R = H. (-)-7-Chloro-10-sulfonamido-1,2,3,4,4a,5,6, 10b-*trans*-octahydro-3N-benzylbenzo[f] isoquinoline (0.20 g, 0.51 mmol), ethanol (50 mL), and palladium hydroxide, 20% on carbon (0.40 g) are shaken under hydrogen (48 PSI) for 6 hours. The mixture is filtered through diatomaceous earth and solvents removed under vacuum to yield a white solid, 0.18 g. Recrystallization from ether and methanol yields a pink solid, 216°C m.p.

Preparation 133(b), Example 17, **(-)-10-Methylsulfonamido-1,2,3,4,4a,5,6,10b-*trans*-octahydrobenzo [f]isoquino-line hydrochloride,** see CHART C, STEP 133, Compound C134, R = $CH_3$. (-)-7-Chloro-10-methylsulfonamido-1,2,3,4,4a,5,6,10b-*trans*-octahydro-3N-benzylbenzo [f]isoquinoline (0.78 g, 1.93mmol), ethanol (50 mL), and palladium hydroxide, 20% on carbon (0.69 g) are shaken under hydrogen (43 PSI) for 21 hours. The mixture is filtered through diatomaceous earth and solvents removed under vacuum to yield a pale foam, m.p. 95°C. $[\alpha]^{25}_{589}$= -148.1° (c= 0.96).

STEP 134

Preparation 134(a), Example 18, **(-)-10-Sulfonamido-1,2,3,4,4a,5,6,10b-*trans*-octahydro-3N-propylbenzo[f]iso-quinoline,** see CHART C, STEP 134, Compound C135, R = H. Bromopropane (0.02 mL, 0.25 mmol) is added to (-)-10-sulfonamido-1,2,3,4,4a,5,6,10b-*trans*-octahydrobenzo[f]isoquinoline hydrochloride (0.077 g, 0.25 mmol) and triethylamine (0.08 mL, 0.56 mmol) in acetonitrile (5 mL) and DMF (2 mL). After heating at 80°C for 4 hours, ether and aqueous sodium carbonate are added. The organic layer is washed with water and brine and dried over sodium sulfate to yield an amber oil.

Preparation 134(b), Example 19, **(-)-10-Methylsulfonamido-1,2,3,4,4a,5,6,10b-*trans*-octahydro-3N-propyl-benzo[f]isoquinoline,** see CHART C, STEP 134, Compound C135, R = $CH_3$. Bromopropane (0.05 mL, 0.50 mmol) is added to (-)-10-methylsulfonamido-1,2,3,4,4a,5,6, 10b-*trans*-octahydrobenzo[f]isoquinoline hydrochloride (0.15 g, 0.50 mmol) and triethylamine (0.14 mL, 1.00 mmol) in acetonitrile (4 mL) and DMF (1 mL). After heating at 50°C for 4 hours, ether and aqueous sodium carbonate are added. The organic layer is washed with water and brine and dried over sodium sulfate to yield a white foam, m.p. 94- 100°C. $[\alpha]^{25}_{589}$= -187.6° (c= 0.47). This foam is dissolved in methanol and ether, then hydrochloric acid in ether is added. The solid is recrystallized from methanol and ether to yield gray crystals, m.p. 299-301 °C.

CHART C begins next page.

CHART C (6-6-6 ring system) page 1

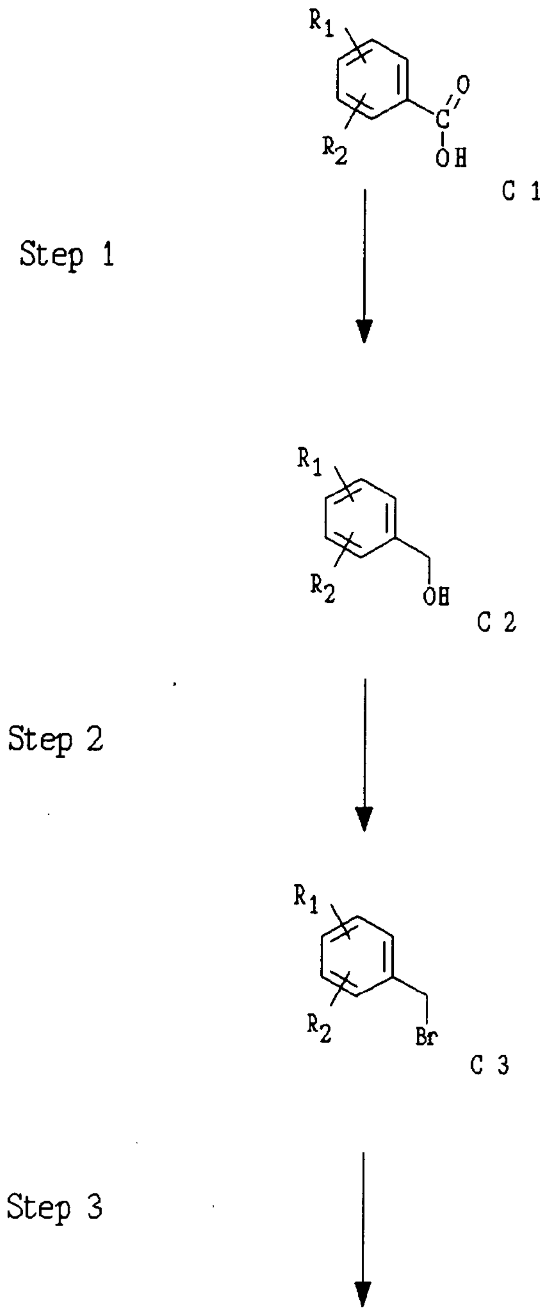


C 1

C 2

C 3

(continued)

CHART C page 2

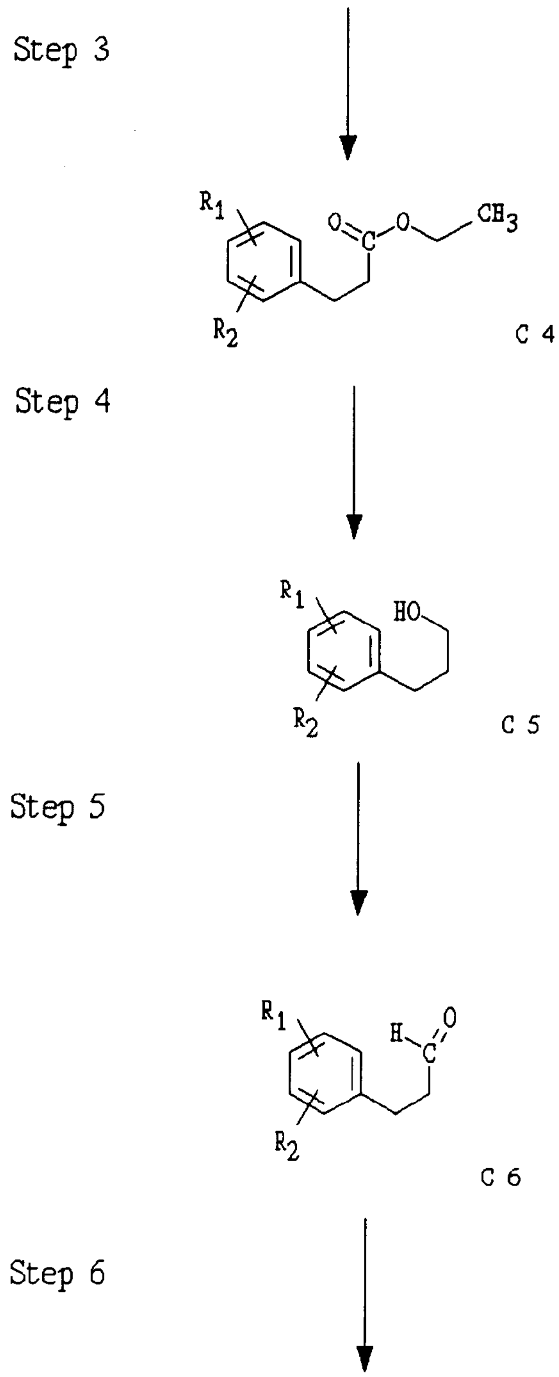


C 4

C 5

C 6

(continued)

CHART C page 3

Step 6

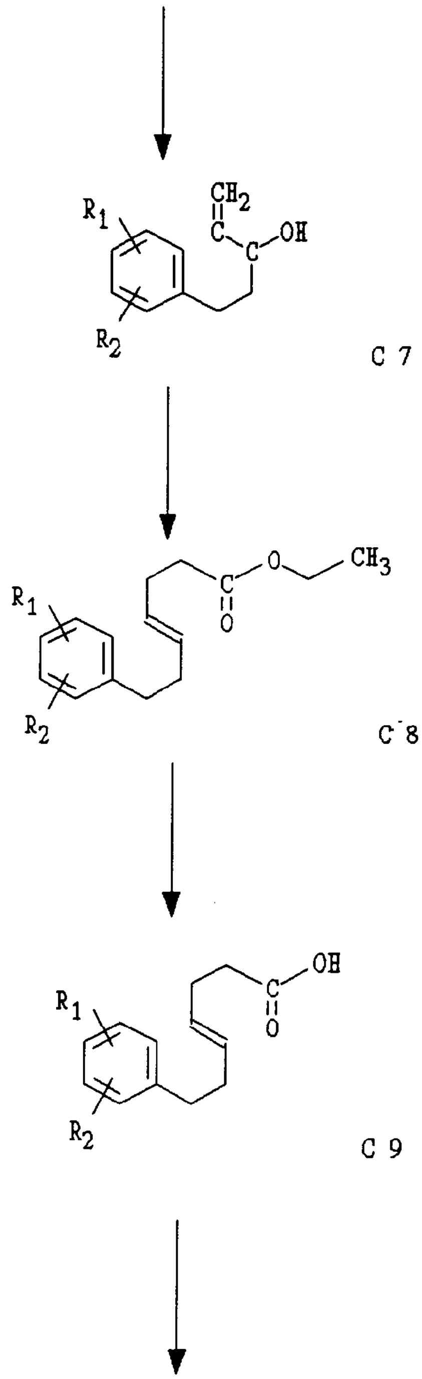

C 7

Step 7

C 8

Step 8

C 9

Step 9

(continued)

CHART C page 4

Step 9

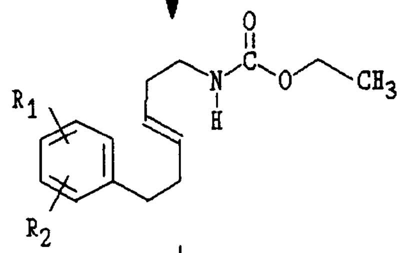

C 10

Step 10

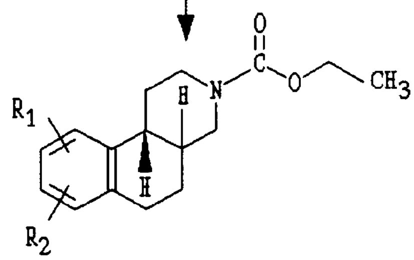

C 11

Step 11

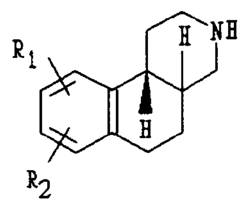

C 12

Step 12

(continued)

CHART C page 5

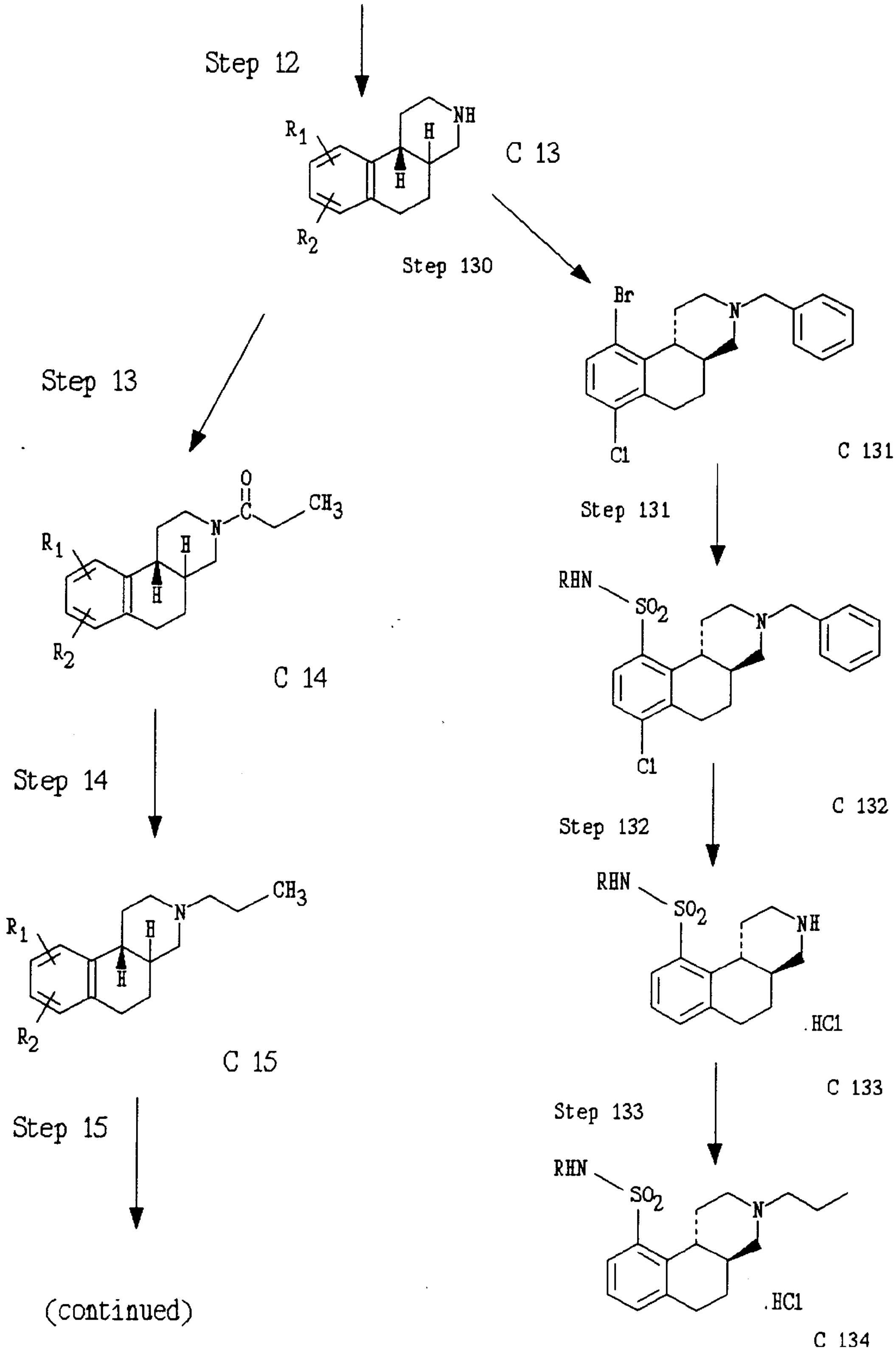


C 13

C 131

C 14

C 132

C 15

C 133

C 134

(continued)

CHART C page 6

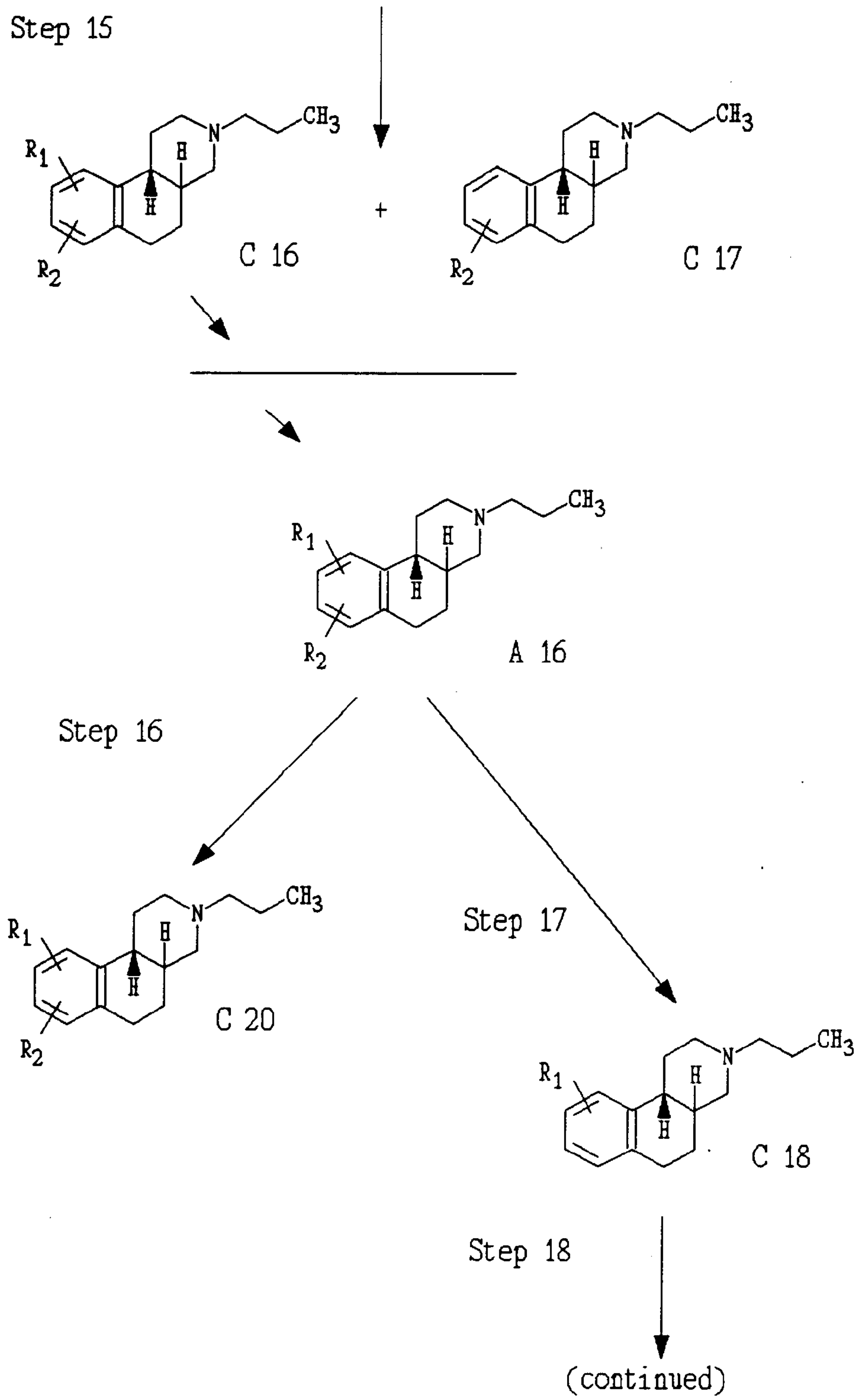


(continued)

CHART C page 7

Step 18

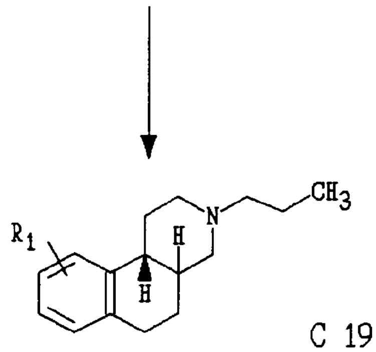

C 19

**Claims**

1. A compound of formula I, below,

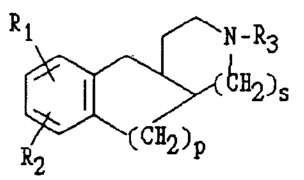

Formula I

wherein p and s are independently related and may be either 1 or 2,
wherein $R_1$ is
- a) -H,
- b) -Halo,
- c) -CN,
- d) $-CO_2H$, $-CO_2R_{1-1}$,
- e) $-CONH_2$, $-CONHR_{1-1}$, $-CON(R_{1-1})_2$,
- g) -SH, $-SR_{1-1}$,
- h) $-SO_2R_{1-1}$,
- i) $-SO_2NH_2$, $-SO_2NHR_{1-1}$ , $-SO_2N(R_{1-1})_2$,
- j) $-OR_{1-1}$
- k) $-OSO_2CF_3$ $-OSO_2R_{1-1}$,
- l) $-NH_2$, $-NHR_{1-1}$, or $-N(R_{1-1})_2$;

wherein $R_{1-1}$ =
- a) -H, except where p = 1 and s = 2
- b) -($C_1$-$C_8$ alkyl),
- c) -($C_1$-$C_8$ alkenyl),
- d) -($C_6$ aryl),
- e) -($C_3$-$C_{10}$ cycloalkyl),
- f) -5 and 6 member heterocyclics,
- g) -($C_1$-$C_8$ alkyl)-(5 or 6 member heterocyclics)

wherein $R_2$ is
- a) -H,
- b) -Halo,
- c) -CN,
- d) $-CF_3$
- e) -SH, or $-SR_{2-1}$;

wherein $R_{2-1}$=

a) -H,
b) -($C_1$-$C_8$ alkyl),
c) -($C_1$-$C_8$ alkenyl),
d) -($C_6$ aryl),
e) -($C_3$-$C_{10}$ cycloalkyl),
f) -5 or 6 member heterocyclics,
g) -($C_1$-$C_8$ alkyl)-(5 or 6 member heterocyclics)

wherein $R_3$ is
a) -H, except where p = 1 and s = 2
b) -($C_1$-$C_8$ alkyl),
c) -($C_1$-$C_8$ alkenyl),
d) -($C_6$ aryl),
e) -($C_3$-$C_{10}$ cycloalkyl),
f) -5 or 6 member heterocyclics,
g) -($C_1$-$C_8$ alkyl)-(5 or 6 member heterocyclics)

or a pharmacologically acceptable salt thereof.

**2.** A compound of claim 1, wherein p = 2 and s = 1.

**3.** A compound of claim 2, wherein $R_1$ is
a) -Halo,
b) -CN,
c) -$CONH_2$, -$CONHR_{1-1}$, or -$CON(R_{1-1})_2$.

**4.** A compound of claim 2, wherein $R_2$ is
a) -Halo,
b) -CN,
c) -$CONH_2$, -$CONHR_{2-1}$, or -$CONR_{2-1}$.

**5.** A compound of claim 2, wherein $R_3$ is
a) -H,
b) -($C_1$-$C_8$) alkyl
c) -($C_1$-$C_8$) alkenyl
d) -($C_3$-$C_{10}$) cycloalkyl,
e) -($C_6$-$C_{12}$ aryl),
f) 5 or 6 member heterocyclics, or a pharmacologically acceptable salt thereof.

**6.** A compound of claim 3, wherein $R_2$ is
a) -Halo,
b) -CN,
c) -$CONH_2$, -$CONHR_{2-1}$, or -$CON(R_{2-1})_2$.

**7.** A compound of claim 6, wherein $R_3$ is
a) -H,
b) -($C_1$-$C_8$) alkyl
c) -($C_1$-$C_8$) alkenyl
d) -($C_3$-$C_{10}$) cycloalkyl,
e) -($C_6$-$C_{12}$ aryl),
f) 5 and 6 member heterocyclics, or a pharmacologically acceptable salt thereof.

**8.** A compound of claim 7 wherein $R_1$ is $CONH_2$.

**9.** A compound of claim 8 selected from,

**(-)-7-Chloro-10-carboxamido-1,2,3,4,4a,5,6,10b-*trans*-octahydro-3N-propylbenzo[f]isoquinoline,**

**(+)-7-Chloro-10-carboxamido-1,2,3,4,4a,5,6,10b-*trans*-octahydro-3N-propylbenzo[f]isoquinoline,**
**(-)-10-Carboxamido-1,2,3,4,4a,5,6,10b-octahydro-*trans*-3N-propylbenzo [f]isoquinoline,** or
**(+)-10-Carboxamido-1,2,3,4,4a,5,6,10b octahydro-*trans*-3N-propylbenzo[f]isoquinoline.**

**10.** A compound of claim 7 wherein $R_1$ is CN.

**11.** A compound of claim 10 selected from
**(-)-7-Chloro-10-cyano-1,2,3,4,4a,5,6,10b-*trans*-octahydro-3N-propylbenzo[f]isoquinoline,**
**(+)-7-Chloro-10-cyano-1,2,3,4,4a,5,6,10b-*trans*-octahydro- 3N-propylbenzo[f]isoquinoline,**
**(-)-10-Cyano-1,2,3,4,4a,5,6,10b-octahydro-*trans*-3N-propylbenzo[f]isoquinoline,** or
**(+)-10-Cyano-1,2,3,4,4a,5,6,10b-octahydro-*trans*-3N-propylbenzo[f]isoquinoline.**

**12.** A compound of claim 7 wherein $R_1$ is Halo, or H.

**13.** A compound of claim 12 selected from,
**(-)-10-Bromo-7-chloro-1,2,3,4,4a,5,6,10b-*trans*-octahydro-3N-propylbenzo[f]isoquinoline,**
**(+)-10-Bromo-7-chloro-1,2,3,4,4a,5,6,10b-*trans*-octahydro-3N-propylbenzo[f]isoquinoline,**
**(-)7-Chloro-1,2,3,4,4a,5,6,10b-*trans*-octahydro-3N-propylbenzo[f]isoquinoline, or**
**(+)7-Chloro-1,2,3,4,4a,5,6,10b-*trans*-octahydro-3N-propylbenzo[f]isoquinoline.**

**14.** A compound of claim 1, wherein p = 1 and s = 2.

**15.** A compound of claim 14,
wherein $R_1$ is
a) -Halo,
b) -CN,
c) $-CONH_2$, $-CONHR_{1-1}$, or $-CON(R_{1-1})_2$.

**16.** A compound of claim 14,
wherein $R_2$ is
a) -Halo, or
b) -CN.

**17.** A compound of claim 14,
wherein $R_3$ is
a) $-(C_1-C_8)$ alkyl
b) $-(C_1-C_8)$ alkenyl
c) $-(C_3-C_{10})$ cycloalkyl,
d) $-(C_6$ aryl),
e) 5 or 6 member heterocyclics.

**18.** A compound of claim 15,
wherein $R_2$ is
a) -Halo, or
b) -CN,
wherein $R_3$ is
a) $-(C_1-C_8)$ alkyl
b) $-(C_1-C_8)$ alkenyl
c) $-(C_3-C_{10})$ cycloalkyl,
d) $-(C_6-C_{12}$ aryl),
e) 5 and 6 member heterocyclics.

**19.** A compound of claim 18 wherein $R_1$ is $-CONH_2$, $-CONHR_{1-1}$, or $-CON(R_{1-1})_2$.

**20.** A compound of claim 19 selected from,
**(+)-trans-10-carboxamido-5a,10b-dihydro-3N-*n*-propyl-6H-indeno[1,2-d]azepine**

**(-)-trans-10-carboxamido-5a,10b-dihydro-3N-*n*-propyl-6H-indeno[1,2-d]azepine**
**(+)-trans-7-carboxamido-5a,10b-dihydro-3N-*n*-propyl-6H-indeno[1,2-d]azepine**
**(-)-trans-7-carboxamido-5a,10b-dihydro-3N-*n*-propyl-6H-indeno[1,2-d]azepine.**

**21.** A compound of claim 18 wherein $R_2$ is CN.

**22.** A compound of claim 21 selected from
**(+)-trans-7-cyano-5a,10b-dihydro-3N-*n*-propyl-6H-indeno[1,2-d]azepine**
**(-)-trans-7-cyano-5a,10b-dihydro-3N-*n*-propyl-6H-indeno[1,2-d]azepine**.

**23.** A compound of claim 18 wherein $R_1$ is $OR_3$, $R_2$ is Cl.

**24.** A compound of claim 23, selected from
cis-7-chloro-10-methoxy-5a,10b-dihydro-3N-n-propyl-6H-indeno [1,2-d]azepine and
trans-7-chloro-10-methoxy-5a,10b-dihydro-3N-n-propyl-6H-indeno [1,2-d]azepine.

**25.** A compound of claim 14, wherein $R_3$ = H.

**26.** A compound of claim 25, selected from
(+)-trans-10-carboxamido-5a,10b-dihydro-6H-indeno[1,2-d]azepine
(-)-trans-10-carboxamido-5a,10b-dihydro-6H-indeno[1,2-d]azepine
(+)-trans-7-carboxamido-5a,10b-dihydro-6H-indeno[1,2-d]azepine
(-)-trans-7-carboxamido-5a,10b-dihydro-6H-indeno[1,2-d]azepine.

**27.** A compound of any of claims 1 to 26, for therapeutic use.

**28.** A pharmaceutical composition comprising a compound of any of claims 1 to 26 and a pharmaceutically-acceptable carrier.

**29.** Use of a compound of any of claims 1 to 26 for the manufacture of a medicament for use in treating central nervous system disorders associated with serotonin and/or dopamine receptor activity.

European Patent Office

# PARTIAL EUROPEAN SEARCH REPORT

which under Rule 45 of the European Patent Convention shall be considered, for the purposes of subsequent proceedings, as the European search report

Application Number: EP 92 30 9695
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | EP-A-0 410 535 (MERCK SHARP AND DOHME LTD)<br>* claims * | 1-8,10, 12,27-29 | C07D221/10<br>C07D223/32<br>C07D221/16<br>C07D223/14<br>A61K31/47<br>A61K31/435 |
| X | EP-A-0 059 553 (SMITHKLINE CORPORATION)<br>* claims * | 1,2,5, 27-29 | |
| D | & US-A-4 341 786 | | |
| X | EP-A-0 106 486 (ELI LILLY AND COMPANY)<br>* claims * | 1,2,5, 27-29 | |
| D | & GB-A-2 126 584 | | |
| D,X | WO-A-9 006 927 (ABBOTT LABORATORIES)<br>* claims * | 1,27-29 | |
| X | FR-A-2 378 015 (SANDOZ S. A.)<br>* claims * | 1,2,3,5, 12,27-29 | |
| D | & GB-A-1 596 170 | | TECHNICAL FIELDS SEARCHED (Int. Cl. 5)<br>C07D |

-/--

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 19 NOVEMBER 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 92 30 9695
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| X | US-A-5 049 564 (JOHN F. DEBERNARDIS)<br>* claims *<br>--- | 1,27-29 | |
| X | CHEMICAL ABSTRACTS, vol. 81, no. 1, 8 July 1974, Columbus, Ohio, US; abstract no. 3750c, A.P.BOYAKHCHYAN ET AL '2,3,4,5,5a,6,7,11b-Octahydro-1H-naphth(1,2-d)azepine' page 303 ;<br>* abstract *<br>& ARM.KHIM ZH. vol. 26, no. 12, pages 1026 - 1029<br>--- | 1,14 | |
| X | CHEMICAL ABSTRACTS, vol. 82, no. 3, 20 January 1975, Columbus, Ohio, US; abstract no. 16693g, A.P.BOYAKHCHYAN ET AL 'Stereoisomeric 1,2,3,4,5,5a,6,10b-octahydroindeno(1,2-c)azepines' page 486 ;<br>* abstract *<br>& KHIM. GETEROTSIKL SOEDIN no. 8, 1974, pages 1129 - 1132<br>--- | 1,14 | TECHNICAL FIELDS SEARCHED (Int. Cl. 5) |
| X | CHEMICAL ABSTRACTS, vol. 115, no. 7, 19 August 1991, Columbus, Ohio, US; abstract no. 64025, A.A HANCOCK ET AL 'Molecular design of novel ligands for 5-HT1a receptors' page 19 ;<br>* abstract *<br>& J. RECEPT.RES. vol. 11, no. 4, 1991, pages 177 - 196<br>---<br>-/-- | 1,27-29 | |

EPO FORM 1503 03.82 (P04E10)

European Patent Office

# PARTIAL EUROPEAN SEARCH REPORT

Application Number
EP 92 30 9695
Page 3

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| X | CHEMICAL ABSTRACTS, vol. 92, no. 15, 14 April 1980, Columbus, Ohio, US; abstract no. 128691t, NARESH KUMAR ET AL 'Agents acting on the central nervous system.Part XXVIII. 2-substituted 1,2,3,4,4a,9,10,10a-octahydrobenzo(f)isoquinolines' page 678 ; * abstract * & INDIAN J. CHEM, SECT B vol. 17-B, no. 3, 1979, page 239-243 --- | 1,2, 27-29 | |
| P,X | EP-A-0 463 691 (MERCK SHARP AND DOHME LTD) * claims * ----- | 1,27-29 | TECHNICAL FIELDS SEARCHED (Int. Cl. 5) |

EPO FORM 1503 03.82 (P04E10)

EP 92 30 9695

-C-

INCOMPLETE SEARCH

Claims searched completely: 9,11,13,20,22,24,26

Claims searched incompletely: 1-8,10,12,14-19,21,23,25,27,28,29

------

There is a discrepancy between the compounds prepared and claimed "per se" (see particularly claims 9,11,13, 20,22,24,26) and the compounds covered by the general formula I of claim 1 which neither covers benzo [f] isoquinoline nor indeno [2-1-c] azepine (According to formula 1 and definition of p, ring B can only have six or seven atoms).
The formula I must probably be read as

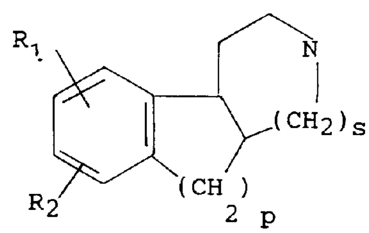

The search has been performed for the compounds of the examples and extended to compounds covered by this modified formula.